# EUROPEAN PATENT APPLICATION

(11) **EP 3 226 238 A1**
(43) Date of publication of application: **04.10.2017**
(21) Application number: 17163034.6
(22) Date of filing: 27.03.2017
(51) Int. Cl.: G10K 11/02, A61B 8/00

(54) **ACOUSTIC MATCHING MATERIAL SUPPLY DEVICE, ULTRASONIC PROBE UNIT, ULTRASONIC MEASUREMENT APPARATUS, AND ULTRASONIC IMAGE DISPLAY**

(30) Priority: 29.03.2016 JP 2016065203
(71) Applicant: Seiko Epson Corporation, Tokyo 160-8801 (JP)
(72) Inventor: Nakazawa, Yusuke, Nagano, 392-8502 (JP)
(74) Representative: Hoffmann Eitle

(57) **Abstract**

An acoustic matching material supply device includes a frame portion that is attached to an ultrasonic probe including an ultrasonic sensor surface for performing at least one of transmission and reception of ultrasonic waves, and ejects an acoustic matching material, in which the frame portion includes an inner peripheral surface included in a plane intersecting the ultrasonic sensor surface, a plurality of ejection ports that are provided on the inner peripheral surface or in a region including the inner peripheral surface, and ejects the acoustic matching material, an introduction port that introduces the acoustic matching material from the outside, and a flow passage through which the ejection ports and the introduction port communicate with each other.

## Description

### BACKGROUND

### 1. Technical Field

The present invention relates to an acoustic matching material supply device, an ultrasonic probe unit, an ultrasonic measurement apparatus, and an ultrasonic image display.

### 2. Related Art

In the related art, in a case where ultrasonic measurement is performed on a target object such as a living body, it is necessary to bring an ultrasonic probe into contact with the target object. At this time, since propagate efficiency of an ultrasonic wave is reduced if an air layer is interposed between the ultrasonic probe and the target object, it is necessary to apply an acoustic matching material having acoustic impedance similar to that of the target object between the ultrasonic probe and the target object. However, if the acoustic matching material becomes insufficient due to drying of the acoustic matching material, it is necessary to stop an ultrasonic process and to supply the acoustic matching material again, and this is complicated work.

In order to eliminate such work, in the related art, there is a device which can automatically supply an acoustic matching material between an ultrasonic probe and a target object (for example, refer to JP-A-2006-6827).

JP-A-2006-6827 discloses an ultrasonic probe attachment which supplies an acoustic matching material to a tip of an ultrasonic probe main body. Specifically, the ultrasonic probe main body is provided with a shaft and a transmission/reception portion, and the transmission/reception portion is provided on a part of a circumferential surface of the shaft along an axial direction. An attachment which is attachable to and detachable from the shaft is provided, and the attachment includes a pipe which has a rectangular shape along the axial direction of the shaft. A tip of the pipe is located near the transmission/reception portion on the tip side of the shaft, and an acoustic matching material supplied from a basal end side of the pipe is supplied to the vicinity of the transmission/reception portion from the tip of the pipe.

However, in the ultrasonic probe attachment disclosed in JP-A-2006-6827, the transmission/reception portion of the ultrasonic probe main body is wider than a supply port of the tip of the pipe, and thus it is hard to supply a sufficient amount of an acoustic matching material to the entire gap between the transmission/reception portion and a living body.

### SUMMARY

Advantage of some aspects of the invention is to provide an acoustic matching material supply device which can supply a sufficient amount of an acoustic matching material to a gap with a target object, an ultrasonic probe unit, an ultrasonic measurement apparatus, and an ultrasonic image display.

An acoustic matching material supply device according to an application example includes a frame portion that is attached to an ultrasonic probe including an ultrasonic sensor surface for performing at least one of transmission and reception of ultrasonic waves, and ejects an acoustic matching material, in which the frame portion includes an inner peripheral surface included in a plane intersecting the ultrasonic sensor surface, an ejection port that is provided on the inner peripheral surface or in a region including the inner peripheral surface, and ejects the acoustic matching material, an introduction port that introduces the acoustic matching material from the outside, and a flow passage through which the ejection port and the introduction port communicate with each other.

In this application example, a plurality of ejection ports are provided on the inner peripheral surface of the frame portion attached to the ultrasonic probe or a region including the inner peripheral surface. The frame portion is provided with the introduction port that introduces the acoustic matching material from the outside, and the flow passage through which the ejection ports and the introduction port communicate with each other.

Thus, if an acoustic matching material is introduced from the introduction port, the acoustic matching material is ejected from each ejection port through the flow passage. Consequently, the acoustic matching material from a plurality of locations is supplied from an outer peripheral side toward the center in a region surrounded by the frame portion with respect to the ultrasonic sensor surface, and thus a sufficient amount of the acoustic matching material can be widely spread on the ultrasonic sensor surface, for example, compared with a case where the acoustic matching material is supplied from a single location. Therefore, it is possible to effectively prevent a reduction in ultrasonic wave propagation efficiency due to intervention of an air layer when an ultrasonic probe is used, and thus to perform ultrasonic measurement using the ultrasonic probe with high accuracy.

In the acoustic matching material supply device according to the application example, it is preferable that a plurality of the frame portions are provided to be connected to each other along the ultrasonic sensor surface.

In the application example with this configuration, a plurality of frame portions are connected to each other along the sensor surface, and flow passages of the respective frame portions are connected to each other. In this configuration, since an acoustic matching material is ejected from respective inner peripheral surfaces of the plurality of frame portions, the acoustic matching material can be widely and uniformly spread with respect to the sensor surface.

In the acoustic matching material supply device according to the application example, it is preferable that the plurality of frame portions are connected to each other in a line along a first direction.

In the application example with this configuration, since a plurality of frame portions are connected to each other in the first direction, an acoustic matching material can be uniformly supplied along the first direction.

In the acoustic matching material supply device according to the application example, it is preferable that the ultrasonic sensor surface has an ultrasonic measurement region, and the frame portion includes an attaching/detaching mechanism that allows the frame portion to be attached to or detached from the ultrasonic probe so that the inner peripheral surface avoids the ultrasonic measurement region.

Meanwhile, as an ultrasonic probe performing transmission and reception of ultrasonic waves, there is a probe in which a plurality of ultrasonic measurement regions are disposed to be arranged in the first direction, and transmission and reception of ultrasonic waves are performed in each ultrasonic measurement region. In the application example with the configuration described above, the attaching/detaching mechanisms for attaching and detaching the frame portions are provided so that each ultrasonic measurement region does not overlap the inner peripheral surface when the acoustic matching material supply device is attached to the ultrasonic probe. Thus, the frame portion does not hinder ultrasonic measurement (transmission and reception of ultrasonic waves) in each ultrasonic measurement region, and a sufficient amount of an acoustic matching material can be supplied to each ultrasonic measurement region.

In the acoustic matching material supply device according to the application example, it is preferable that, among the plurality of frame portions, a frame portion located at the center in a first direction has a smaller width dimension along the first direction than a width dimension of a frame portion disposed at an end in the first direction.

As an ultrasonic probe, there is a so-called convex type ultrasonic probe in which a measurer tightly presses an ultrasonic sensor surface against a target object, and performs ultrasonic measurement while changing an attitude of the ultrasonic probe or changing a position of tightly pressing the ultrasonic sensor surface in order to search for a measurement location. In such an ultrasonic probe, as ultrasonic measurement is performed closer to a central position, a measurer force is applied, and thus an acoustic matching material is likely to be reduced. In the application example with the configuration described above, a width dimension of a frame portion (first frame portion) disposed at the center in the first direction is smaller than a width dimension of a frame portion (second frame portion) disposed at an end in the first direction. In other words, the first frame portion has a small interval between inner peripheral surfaces opposing each other. Consequently, even in a case where the same amount of an acoustic matching material is supplied to a region surrounded by the first frame portion and a region surrounded by the second frame portion, a larger amount of the acoustic matching material per unit area (per unit volume) is supplied to the region surrounded by the first frame portion. In other words, a larger amount of an acoustic matching material is supplied to the center in which an amount of the acoustic matching material is easily reduced due to movement of an ultrasonic probe or an attitude change thereof. Consequently, it is possible to prevent deterioration in ultrasonic measurement accuracy or ultrasonic measurement efficiency due to a reduction in an acoustic matching material.

In the acoustic matching material supply device according to the application example, it is preferable that the plurality of frame portions are provided in an array form along a first direction and a second direction intersecting the first direction.

As an ultrasonic probe, there is not only the ultrasonic probe having a configuration in which a plurality of ultrasonic measurement regions are disposed in the first direction as described above, but also a probe having a configuration in which a plurality of ultrasonic measurement regions are disposed in a two-dimensional array form. In the application example with the configuration described above, since a plurality of frame portions are connected to each other in an array form along the first direction and the second direction with respect to such an ultrasonic probe, it is possible to appropriately supply an acoustic matching material to each of ultrasonic measurement regions arranged in a two-dimensional array form.

In the acoustic matching material supply device according to the application example, it is preferable that the plurality of frame portions include attaching/detaching mechanisms that allow the frame portion to be attached to or detached from the ultrasonic probe so that the inner peripheral surfaces avoid a plurality of ultrasonic measurement regions with respect to the ultrasonic sensor surface in which the ultrasonic measurement regions are disposed in an array form along the first direction and the second direction.

In the application example with this configuration, with respect to ultrasonic measurement regions configured in the above-described two-dimensional array structure, the frame portions do not hinder ultrasonic measurement in each ultrasonic measurement region, and a sufficient amount of an acoustic matching material can be supplied to each ultrasonic measurement region.

In the acoustic matching material supply device according to the application example, it is preferable that, among the plurality of frame portions, a frame portion located at the center has a smaller width dimension along at least one of the first direction and the second direction than a width dimension of a frame portion disposed at an outer periphery.

In the application example with this configuration, in a case where the above-described non-fixation type (convex type) ultrasonic probe is used as an ultrasonic probe, it is possible to supply a sufficient amount of an acoustic matching material to the center of the ultrasonic probe in which the acoustic matching material is easily reduced compared with the outer periphery, and thus to prevent deterioration in ultrasonic measurement accuracy or ultrasonic measurement efficiency due to a reduction in an acoustic matching material.

In the acoustic matching material supply device according to the application example, it is preferable that the frame portion includes a protrusion that protrudes from the inner peripheral surface toward the center and has a branching passage communicating with the flow passage, and the protrusion is provided with a second ejection port that communicates with the branching passage and ejects the acoustic matching material to the outside on a side surface which intersects the ultrasonic sensor surface and faces the ultrasonic sensor surface.

In the application example with this configuration, an acoustic matching material can be supplied from the second ejection port provided at the protrusion toward the center of the ultrasonic sensor surface. Thus, for example, compared with a case where an acoustic matching material is supplied from the outside of the ultrasonic sensor surface, an acoustic matching material can be efficiently and widely spread to the entire ultrasonic sensor surface, and thus the acoustic matching material can be uniformly supplied to the ultrasonic sensor surface.

It is preferable that the acoustic matching material supply device according to the application example further includes a deaeration passage that discharges a gas contained in an ejected acoustic matching material which is ejected from the frame portion, to the outside.

If an acoustic matching material is supplied to a region surrounded by the frame portion, a gas contained in the acoustic matching material may remain. In contrast, in the application example with the configuration described above, since the gas is discharged from the deaeration passage, it is possible to prevent a problem that a gas remains in an acoustic matching material, and thus to prevent deterioration in ultrasonic measurement accuracy.

In the acoustic matching material supply device according to the application example, it is preferable that the frame portion is provided with a notch portion formed by notching a part of the frame portion, and the deaeration passage is provided at the notch portion.

In the application example with this configuration, the notch portion is provided at a part of the frame portion, and the deaeration passage is provided in the notch portion. Thus, it is possible to discharge a gas contained in an acoustic matching material with a simple configuration.

It is preferable that the acoustic matching material supply device according to the application example further includes an opening/closing portion that opens and closes a flow passage between the ejection port and the introduction port.

In the application example with this configuration, a flow passage between the ejection port and the introduction port can be opened and closed by using the opening/closing portion. Thus, the supply of an acoustic matching material can be stopped by closing the flow passage with the opening/closing portion, and the acoustic matching material can be supplied to the ultrasonic sensor surface by opening the opening/closing portion. In other words, it is possible to control the supply of an acoustic matching material with a simple configuration.

It is preferable that the acoustic matching material supply device according to the application example further includes a control unit that controls the opening/closing portion, and the control unit includes a region detection portion that detects an insufficiency region in which the acoustic matching material is insufficient, and a sufficiency region in which the acoustic matching material is sufficient, and an opening/closing control portion that opens the opening/closing portion corresponding to an ejection port provided in the insufficiency region, and closes the opening/closing portion corresponding to an ejection port provided in the sufficiency region.

In the application example with this configuration, the region detection portion detects an insufficiency region in which an acoustic matching material is insufficient, and the opening/closing control portion controls opening and closing of the opening/closing portion so that the acoustic matching material is supplied to the insufficiency region, and the supply of the acoustic matching material to the sufficiency region is stopped. Consequently, it is possible to uniformly supply an acoustic matching material to the ultrasonic sensor surface.

In the acoustic matching material supply device according to the application example, it is preferable that the region detection portion detects the insufficiency region and the sufficiency region on the basis of a received signal acquired by transmitting the ultrasonic waves to a target object from the ultrasonic probe and receiving the ultrasonic waves which are reflected from the target object.

In the application example with this configuration, the region detection portion detects an insufficiency region and a sufficiency region on the basis of ultrasonic wave transmission and reception results using the ultrasonic probe. In other words, since a gas is mixed into an acoustic matching material in the insufficiency region, if transmission and reception of ultrasonic waves are performed, the ultrasonic waves are reflected at an interface between the gas and the acoustic matching material, and the intensity of a received signal increases. On the other hand, since ultrasonic waves propagate into a living body through the acoustic matching material in the sufficiency region, a reception intensity is low, and a timing of detecting a received signal is delayed. Therefore, it is possible to easily determine an insufficiency region and a sufficiency region by performing transmission and reception of ultrasonic waves.

An acoustic matching material supply device according to an application example includes a frame portion that is attached to an ultrasonic probe including an ultrasonic sensor surface for performing at least one of transmission and reception of ultrasonic waves, and ejects an acoustic matching material, in which the frame portion includes a flow passage forming portion in which a flow passage of an acoustic matching material is formed, and an introduction port that introduces the acoustic matching material into the flow passage of the flow passage forming portion, and in which the flow passage forming portion has a central extending portion which extends toward the center of the ultrasonic sensor surface, and the central extending portion is provided with a central ejection port which ejects the acoustic matching material toward the center of the ultrasonic sensor surface.

In this application example, the frame portion is provided with the flow passage forming portion to which an acoustic matching material is supplied from the introduction port, and the flow passage forming portion has the central extending portion which extends toward the center of the ultrasonic sensor surface. The central extending portion is provided with the central ejection port which ejects the acoustic matching material toward the ultrasonic sensor surface. In this configuration, since an acoustic matching material is ejected to the center of the ultrasonic sensor surface from the central ejection port provided at the central extending portion, the acoustic matching material is supplied from the center of the ultrasonic sensor surface toward an outer periphery. In this configuration, for example, compared with a case where an acoustic matching material is supplied from an outer periphery of the ultrasonic sensor surface, it is possible to efficiently and uniformly supply the acoustic matching material to a wide region of the ultrasonic sensor surface.

An ultrasonic probe unit according to an application example includes the acoustic matching material supply device; and the ultrasonic probe.

In this application example, it is possible to efficiently and uniformly supply an acoustic matching material to the ultrasonic sensor surface of the ultrasonic probe. Consequently, when ultrasonic measurement using an ultrasonic probe is performed, it is possible to save time and effort to repeatedly supply and apply an acoustic matching material for a plurality of number of times, and thus to perform highly accurate ultrasonic measurement.

An ultrasonic measurement apparatus according to an application example includes the ultrasonic probe unit; and a control device that controls the ultrasonic probe.

In this application example, it is possible to perform ultrasonic measurement using the ultrasonic probe by controlling the ultrasonic probe with the control device. In this case, as described above, since it is possible to efficiently and uniformly supply the acoustic matching material to a wide region of the ultrasonic sensor surface with the acoustic matching material supply device, in a case where ultrasonic measurement is performed by using the ultrasonic measurement apparatus, for example, it is possible to save time and effort to repeatedly apply an acoustic matching material for a plurality of number of times, and thus to perform highly accurate ultrasonic measurement. Since an acoustic matching material can be uniformly supplied to the ultrasonic sensor surface, it is possible to prevent deterioration in measurement accuracy due to a gas between the ultrasonic probe and a target object, and thus to improve ultrasonic measurement accuracy.

An ultrasonic image display according to an application example includes the ultrasonic measurement apparatus; and a display device that displays an image, in which the control device generates an internal tomographic image of a target object on the basis of ultrasonic measurement using the ultrasonic probe, and displays the ultrasonic image on the display device.

In this application example, since the accuracy of ultrasonic measurement performed by the control device is improved, it is possible to acquire a highly accurate ultrasonic image (an internal tomographic image of a target object) based on results of the ultrasonic measurement, and thus to display the ultrasonic image on the display device. The ultrasonic image is displayed on the display device, and thus it is possible to easily visually recognize whether or not an acoustic matching material is appropriately supplied by the acoustic matching material supply device.

### BRIEF DESCRIPTION OF THE DRAWINGS

The invention will be described with reference to the accompanying drawings, wherein like numbers reference like elements.
Fig. 1 is a diagram illustrating a schematic configuration of an ultrasonic image display according to a first embodiment.
Fig. 2 is a perspective view illustrating a schematic configuration of an ultrasonic probe unit according to the first embodiment.
Fig. 3 is a sectional view illustrating a schematic configuration of an ultrasonic probe according to the first embodiment.
Fig. 4 is a plan view illustrating a schematic configuration of an ultrasonic device according to the first embodiment.
Fig. 5 is a sectional view of the ultrasonic device taken along the line A-A in Fig. 4.
Fig. 6 is a plan view illustrating a schematic configuration of a coupler according to the first embodiment.
Fig. 7 is a schematic sectional view illustrating a configuration of a part of the coupler according to the first embodiment.
Fig. 8 is a sectional perspective view illustrating a schematic configuration of the vicinity of an ejection port according to the first embodiment.
Fig. 9 is a sectional view taken along the line B-B in Fig. 8.
Fig. 10 is a sectional view taken along the line C-C in Fig. 8.
Fig. 11 is a diagram illustrating a schematic configuration of a connector portion according to the first embodiment.
Fig. 12 is a flowchart illustrating a checking process of checking a supplying amount of an acoustic matching material according to the first embodiment.
Fig. 13 is a diagram illustrating an example of an ultrasonic image in a case where an amount of the supplied acoustic matching material is sufficient.
Fig. 14 is a diagram illustrating an example of an ultrasonic image in a case where an amount of the supplied acoustic matching material is insufficient.
Fig. 15 is a sectional view illustrating a case where the ultrasonic probe is fixed to a living body without being attached with the coupler according to the first embodiment.
Fig. 16 is a sectional view illustrating a case where the ultrasonic probe is fixed to a living body in a state of being attached with the coupler according to the first embodiment.
Fig. 17 is a diagram illustrating comparison between thickness dimensions of an acoustic matching material according to the presence or absence of the coupler.
Fig. 18 is a diagram illustrating a relationship between a thickness dimension of the acoustic matching material and the intensity of a received signal based on a reflected ultrasonic wave at an interface position.
Fig. 19 is a diagram illustrating a reduction amount of an acoustic matching material over time.
Fig. 20 is a flowchart illustrating a method of supplying an acoustic matching material according to the first embodiment.
Fig. 21 is a diagram illustrating a configuration of a supply flow passage of the coupler according to a second embodiment.
Fig. 22 is a perspective view illustrating a configuration of the vicinity of a discharge port according to the second embodiment.
Fig. 23 is a block diagram illustrating a functional configuration of a control device according to the second embodiment.
Fig. 24 is a diagram illustrating an example of an ultrasonic image in a case where an acoustic matching material is insufficient in a partial region on an acoustic lens.
Fig. 25 is a diagram illustrating a configuration of a supply flow passage of a coupler according to a third embodiment.
Fig. 26 is a diagram illustrating a configuration of a supply flow passage of a coupler according to a fourth embodiment.
Fig. 27 is a diagram illustrating schematic configurations of a convex type ultrasonic probe and a coupler which is attachably and detachably attached to the ultrasonic probe according to a fifth embodiment.
Fig. 28 is a plan view of the coupler according to the fifth embodiment.
Fig. 29 is a diagram illustrating a thickness dimension of an acoustic matching material in a case where the convex type ultrasonic probe is used.
Fig. 30 is a plan view of a coupler according to a sixth embodiment.
Fig. 31 is a diagram illustrating a schematic configuration of a supply flow passage in a modification example of the first embodiment.
Fig. 32 is a diagram illustrating a schematic configuration of a supply flow passage in a modification example of the second embodiment.

### DESCRIPTION OF EXEMPLARY EMBODIMENTS

### First Embodiment

Hereinafter, a description will be made of a first embodiment according to the invention.

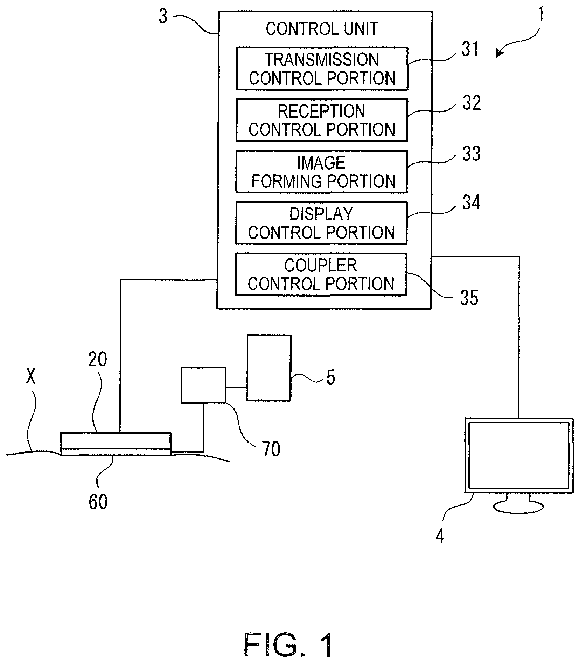

Fig. 1 is a diagram illustrating a schematic configuration of an ultrasonic image display 1 according to the first embodiment.

As illustrated in Fig. 1, the ultrasonic image display 1 of the present embodiment includes an ultrasonic probe unit 2, a control device 3 (control unit) which controls the ultrasonic probe unit 2 , a display device 4 , and a cartridge tank 5 which stores an acoustic matching material. Here, the ultrasonic probe unit 2 and the control device 3 form an ultrasonic measurement apparatus.

The ultrasonic image display 1 sends ultrasonic waves into a living body from the ultrasonic probe unit 2 in a state in which the ultrasonic probe unit 2 is brought into contact with a surface of the living body (target object). Ultrasonic waves reflected from an organ in the living body are received by the ultrasonic probe unit 2, for example, an internal tomographic image of the living body is acquired on the basis of a received signal, and the acquired internal tomographic image is displayed on the display device 4.

The ultrasonic probe unit 2 of the present embodiment is configured to include, as illustrated in Fig. 1, an ultrasonic probe 20 and a coupler 60 which is attachably and detachably attached to ultrasonic probe 20. The coupler 60 is an acoustic matching material supply device which connects the ultrasonic probe 20 to a living body, and supplies an acoustic matching material supplied from the cartridge tank 5 to a gap between the ultrasonic probe 20 and the living body so that the gap is filled with the acoustic matching material.

### Ultrasonic Probe Unit

### Ultrasonic Probe

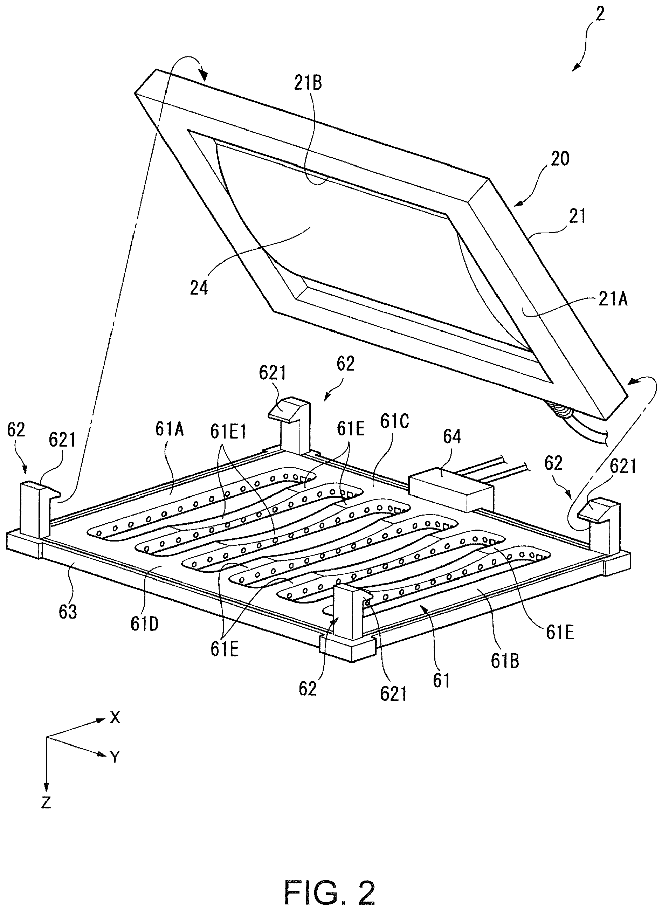

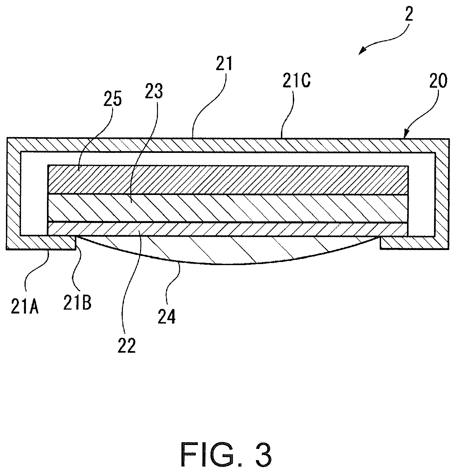

Fig. 2 is a perspective view illustrating a schematic configuration of the ultrasonic probe unit 2. Fig. 3 is a sectional view illustrating a schematic configuration of the ultrasonic probe 20.

As illustrated in Fig. 3, the ultrasonic probe 20 forming the ultrasonic probe unit 2 includes a casing 21, an ultrasonic device 22 provided in the casing 21, a sealing plate 23, an acoustic lens 24, and a circuit board 25 on which a driver circuit and the like for controlling the ultrasonic device 22 are provided.

As illustrated in Fig. 2, the casing 21 is formed, for example, in a rectangular box shape, and one surface side thereof is a measurement surface 21A facing a living body. The measurement surface 21A is provided with a sensor window 21B, and the acoustic lens 24 connected to the ultrasonic device 22 is exposed from the sensor window 21B. The acoustic lens 24 exposed from the sensor window 21B has a cylindrical shape in which a surface shape of a section with respect to an X direction (second direction) is a circular arc. The surface of the acoustic lens 24 is an ultrasonic sensor surface through which ultrasonic waves transmitted and received by the ultrasonic probe 20 pass.

A cable is inserted into a part of the casing 21, and the circuit board 25 of the ultrasonic probe 20 is communicably connected to the control device 3 by the cable.

### Configuration of Ultrasonic Device

Next, a configuration of the ultrasonic device 22 will be described in detail.

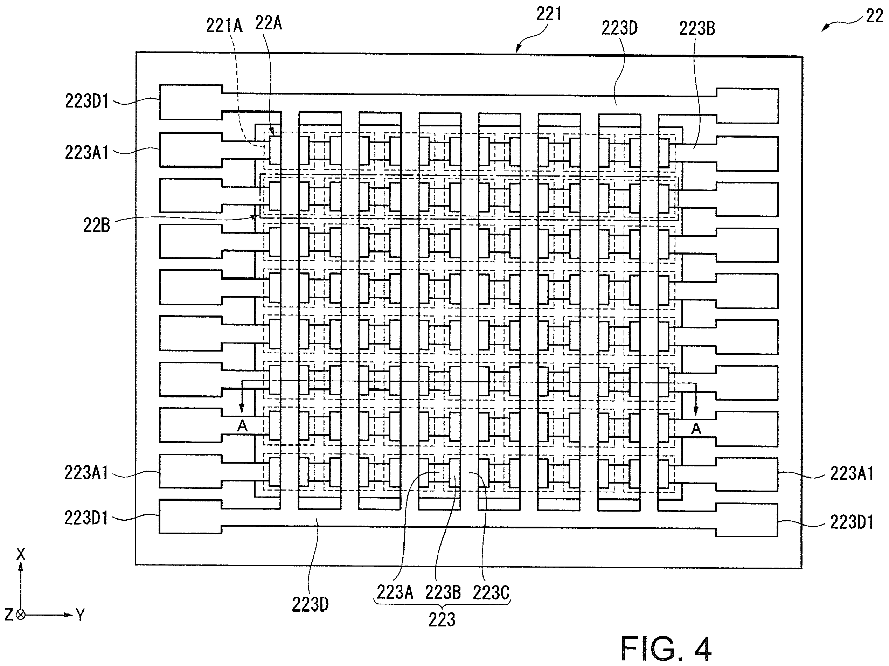

Fig. 4 is a plan view illustrating a schematic configuration of the ultrasonic device 22.

As illustrated in Fig. 4, a plurality of ultrasonic transducers 22A are disposed in a two-dimensional array form in the ultrasonic device 22 along an X direction (a second direction: a slice direction) and a Y direction (a first direction: a scan direction) which intersect (in the present embodiment, exemplarily, orthogonal to) each other. Here, a transmission/reception column 22B (ultrasonic wave measurement region) of one channel is formed of a plurality of ultrasonic transducers 22A disposed in the X direction, and the transmission/reception column of one channel (CH) is arranged in plurality along the Y direction, so that the ultrasonic device 22 with a one-dimensional array structure is formed. For convenience of description, Fig. 4 illustrates a less number of arranged ultrasonic transducers 22A, but, actually, a larger number of ultrasonic transducers 22A are disposed.

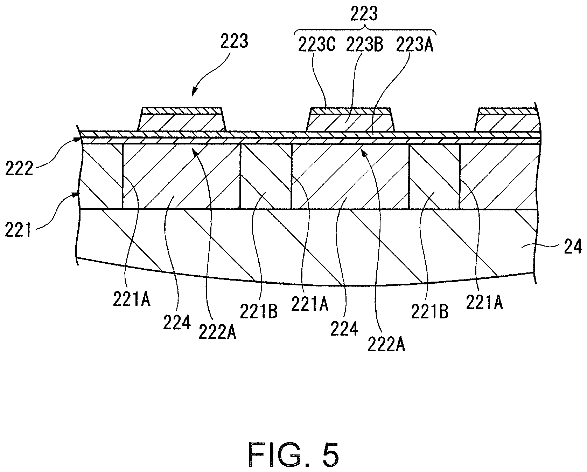

Fig. 5 is a sectional view of the ultrasonic device 22 taken along the line A-A in Fig. 4.

As illustrated in Fig. 5, the ultrasonic device 22 is configured to include an element board 221, a support film 222 laminated on the element board 221, and a piezoelectric element 223 laminated on the support film 222.

The element board 221 is formed of, for example, a semiconductor substrate such as Si. The element board 221 is provided with openings 221A respectively corresponding to the ultrasonic transducers 22A. In the present embodiment, each opening 221A is a through hole which penetrates through the element board 221 in a board thickness direction, and the support film 222 is provided on one end side (sealing plate 23 side) of the through hole.

An acoustic matching layer 224 fills the opening 221A on a side where the support film 222 is not provided, and the acoustic lens 24 is provided via the acoustic matching layer 224.

The support film 222 is made of, for example, SiO₂ or a laminate of SiO₂ and ZrO₂ and is provided to entirely cover the element board 221 on the sealing plate 23 side. In other words, the support film 222 is supported by partition walls 221B forming the opening 221A, and closes the opening 221A on the sealing plate 23 side. A thickness dimension of the support film 222 is sufficiently smaller than a thickness dimension of the element board 221.

The piezoelectric element 223 is provided on the support film 222 closing each of the openings 221A. The piezoelectric element 223 is formed of, for example, a laminate of a lower electrode 223A, a piezoelectric film 223B, and an upper electrode 223C from the support film 222 side.

Here, a portion of the support film 222 closing the opening 221A forms a vibration portion 222A, and a single ultrasonic transducer 22A is formed of the vibration portion 222A and the piezoelectric element 223.

In the ultrasonic transducer 22A, a rectangular wave voltage with a predetermined frequency is applied between the lower electrode 223A and the upper electrode 223C so that the piezoelectric film 223B is bent so as to cause the vibration portion 222A to vibrate, and thus an ultrasonic wave is sent. If the vibration portion 222A vibrates due to an ultrasonic wave reflected from the living body, a potential difference occurs between an upper part and a lower part of the piezoelectric film 223B. Therefore, the received ultrasonic wave can be detected by detecting the potential difference occurring between the lower electrode 223A and the upper electrode 223C.

In the present embodiment, the lower electrode 223A is formed in a linear shape along the X direction, and connects a plurality of ultrasonic transducers 22A forming the transmission/reception column 22B of one channel to each other. Signal terminal portions 223A1 which are electrically connected to the circuit board 25 are provided at both ends (ends on ±X sides) of the lower electrode 223A.

The upper electrode 223C is formed in a linear shape along the Y direction, and connects the ultrasonic transducers 22A arranged in the Y direction to each other. Ends of the upper electrode 223C on ±Y side are connected to common electrode lines 223D. Each of the common electrode lines 223D connects a plurality of upper electrodes 223C disposed along the X direction to each other, and common terminal portions 223D1 which are electrically connected to the circuit board 25 are provided at ends thereof.

### Configuration of Sealing Plate

A planar shape of the sealing plate 23 viewed from the thickness direction is formed to be the same as, for example, that of the element board 221, and reinforces the element board 221. A material or a thickness of the sealing plate 23 influences frequency characteristics of the ultrasonic transducer 22A, and is thus preferably set on the basis of a center frequency of an ultrasonic wave which is transmitted and received in the ultrasonic transducer 22A.

The sealing plate 23 is provided with openings (not illustrated) at positions opposing the signal terminal portions 223A1 and the common terminal portions 223D1 of the element board 221, and thus the signal terminal portions 223A1 and the common terminal portions 223D1 are electrically connected to the circuit board 25 via, for example, an FPC through the openings.

### Configuration of Acoustic Lens

The acoustic lens 24 is provided on the ultrasonic device 22 on an opposite side to the sealing plate 23 via the acoustic matching layer 224. The acoustic lens 24 is exposed to the outside from the sensor window 21B of the casing 21 as illustrated in Fig. 2. As described above, the acoustic lens 24 has a circular shape as a sectional surface shape with respect to the X direction, and thus has a cylindrical shape with the Y direction as an axis. Consequently, ultrasonic waves transmitted from the transmission/reception column 22B disposed along the X direction have a uniform beam shape so as to converge at a focal point position at a predetermined depth in a living body due to the acoustic lens 24.

### Configuration of Circuit Board

The circuit board 25 is provided with a driver circuit and the like for controlling the ultrasonic device 22. The circuit board 25 is provided with, for example, a transmission circuit which performs a process of transmitting an ultrasonic wave from the ultrasonic device 22, a reception circuit which performs a process of receiving the ultrasonic wave based on a received signal output from the ultrasonic device 22, and a switching circuit which switches between connections of each ultrasonic device 22 to the transmission circuit and the reception circuit.

In transmission of an ultrasonic wave, the transmission circuit outputs a transmission signal for transmitting an ultrasonic wave to the ultrasonic device 22 under the control of the control device 3.

In reception of an ultrasonic wave, the reception circuit outputs a received signal which is input from the ultrasonic device 22, to the control device 3. The reception circuit is configured to include, for example, a low noise amplification circuit, a voltage controlled attenuator, a programmable gain amplifier, a low-pass filter, and an A/D converter, and performs signal processes such as conversion of the received signal to a digital signal, removal of a noise component, and amplification to a desired signal level, and outputs the received signal having undergone the processes to the control device 3.

### Coupler

Next, a description will be made of the coupler 60 which is attachably and detachably attached to the ultrasonic probe 20 and forms the ultrasonic probe unit 2 along with the ultrasonic probe 20.

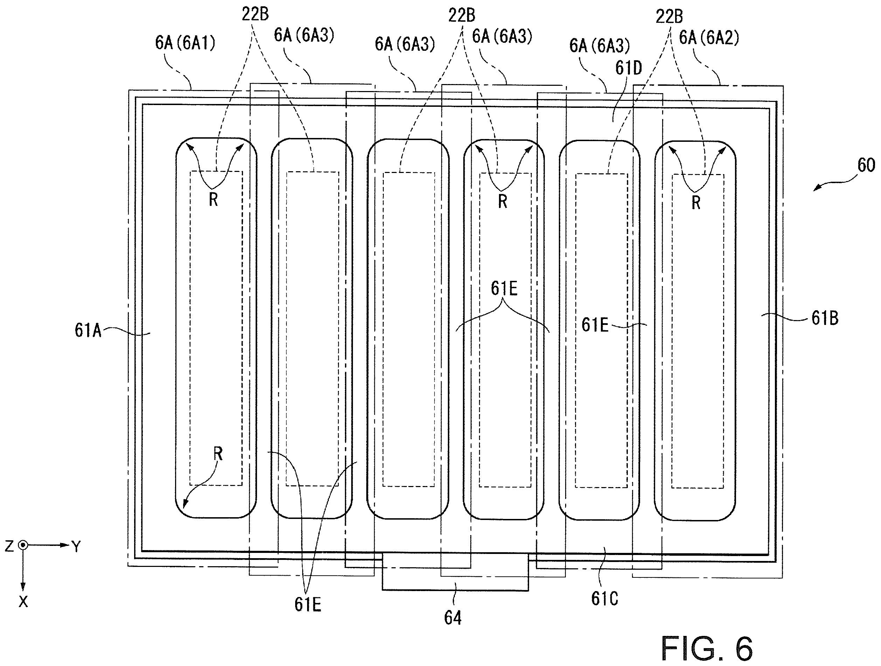

Fig. 6 is a plan view illustrating a schematic configuration of the coupler 60 of the present embodiment.

The coupler 60 is made of a plastic resin such as vinyl chloride or an acrylic resin. As illustrated in Figs. 2 and 6, the coupler 60 includes a frame body 61, an attaching/detaching mechanism 62 which attaches and detaches the coupler 60 to and from the casing 21 of the ultrasonic probe 20, a deaeration portion 63 which is provided over a peripheral direction along an outer peripheral surface of the frame body 61, and a connector portion 64 which is provided on an outer peripheral surface of the frame body 61 and is connected to the cartridge tank 5.

The frame body 61 is formed in a planar rectangular shape which is substantially the same as that of the measurement surface 21A of the casing 21 of the ultrasonic probe 20. The frame body 61 includes a first support portion 61A and a second support portion 61B which are disposed along the X direction, a third support portion 61C and a fourth support portion 61D which connect ends of the first support portion 61A and the second support portion 61B to each other and are also disposed along the Y direction, and a plurality of crossbeams 61E which are crosslinked between the third support portion 61C and the fourth support portion 61D and are provided along the X direction.

In other words, in the coupler 60 of the present embodiment, as illustrated in Fig. 6, a single frame portion 6A (frame portion 6A1) is formed of the first support portion 61A, the crossbeam 61E adjacent to the first support portion 61A on the +Y side, the third support portion 61C, and the fourth support portion 61D. A single frame portion 6A (frame portion 6A2) is formed of the second support portion 61B, the crossbeam 61E adjacent to the second support portion 61B on the -Y side, the third support portion 61C, and the fourth support portion 61D. A single frame portion 6A (frame portion 6A3) is formed of the crossbeams 61E adjacent to each other, the third support portion 61C, and the fourth support portion 61D.

A plurality of frame portions 6A are arranged in a line along the Y direction, and the frame portions 6A are configured to be connected to each other. In the present embodiment, each of the frame portions 6A is disposed to correspond to the transmission/reception column 22B of one channel. When the coupler 60 is attached to the casing 21 and is viewed from a normal direction to the measurement surface 21A, as illustrated in Fig. 6, each crossbeam 61E is disposed at a position not overlapping the ultrasonic transducer 22A between the transmission/reception columns 22B. The transmission/reception column 22B is disposed in the frame inside surrounded by an inner peripheral surface of each frame portion 6A. As will be described later in detail, the frame portion 6A is provided with a plurality of ejection ports 66 on an inner peripheral surface intersecting the measurement surface 21A, and an acoustic matching material is ejected onto the acoustic lens 24 which is an ultrasonic sensor surface from the ejection ports 66 when the coupler 60 is attached to the casing 21.

Each of corners of the inner peripheral surface of the frame portion 6A is a curved surface R having a predetermined diameter dimension. In other words, a corner of an inner peripheral surface of a portion at which the first support portion 61A is connected to the third support portion 61C, a corner of an inner peripheral surface of a portion at which the first support portion 61A is connected to the fourth support portion 61D, a corner of an inner peripheral surface of a portion at which the second support portion 61B is connected to the third support portion 61C, a corner of an inner peripheral surface of a portion at which the second support portion 61B is connected to the fourth support portion 61D, corners between the third support portion 61C and the respective crossbeams 61E, and corners between the fourth support portion 61D and the respective crossbeams 61E are formed so that inner peripheral sides thereof facing the transmission/reception columns 22B are curved surfaces R. Consequently, as described above, when an acoustic matching material is ejected onto the acoustic lens 24, the acoustic matching material easily fills the each corner, and a gas can be prevented from staying.

Here, a distance of the inner peripheral surface between the first support portion 61A and the second support portion 61B is the same as (or slightly larger than) a distance of the acoustic lens 24 along the Y direction, and the acoustic lens 24 is interposed between an inner peripheral surface 61A1 (a surface on the +Y side) of the first support portion 61A and an inner peripheral surface 61B1 (a surface on the -Y side) of the second support portion 61B so that the coupler 60 in the Y direction can be positioned at the casing 21.

The frame body 61 is provided with, for example, the attaching/detaching mechanisms 62 on outer surfaces on the ±X sides. Each of the attaching/detaching mechanisms 62 has a claw 621 protruding toward the casing 21 side. The claws 621 are engaged with a top surface 21C on an opposite side to the measurement surface 21A of the casing 21, and thus a position of the coupler 60 in the X direction can be determined, and the coupler 60 can also be attached to the casing 21.

A side surface of the first support portion 61A on the +Y side, a side surface of the second support portion 61B on the -Y side, and side surfaces of the respective crossbeams 61E on the ±Y sides are flat surfaces along the X direction, and interfaces between the flat surfaces and the above-described curved surfaces R are located outside (±X sides) of at least the acoustic lens 24.

In the crossbeams 61E, lens facing surfaces 61E1 on the -Z side (refer to Fig. 2) facing the measurement surface 21A have circular-arch curved surfaces corresponding to the surface shape (cylindrical shape) of the acoustic lens 24.

Therefore, when the coupler 60 is attached to the casing 21, interference of the curved surfaces R continuing to the first support portion 61A and the second support portion 61B with the acoustic lens 24 or interference of the crossbeams 61E with the acoustic lens 24 is prevented, and thus the coupler 60 can be brought into close contact with and attached to the casing 21. There may be a configuration in which a water-resistant adhesive layer or the like is provided on the coupler 60 facing the casing 21, and thus a gap between the coupler 60 and the casing can be more reliably filled.

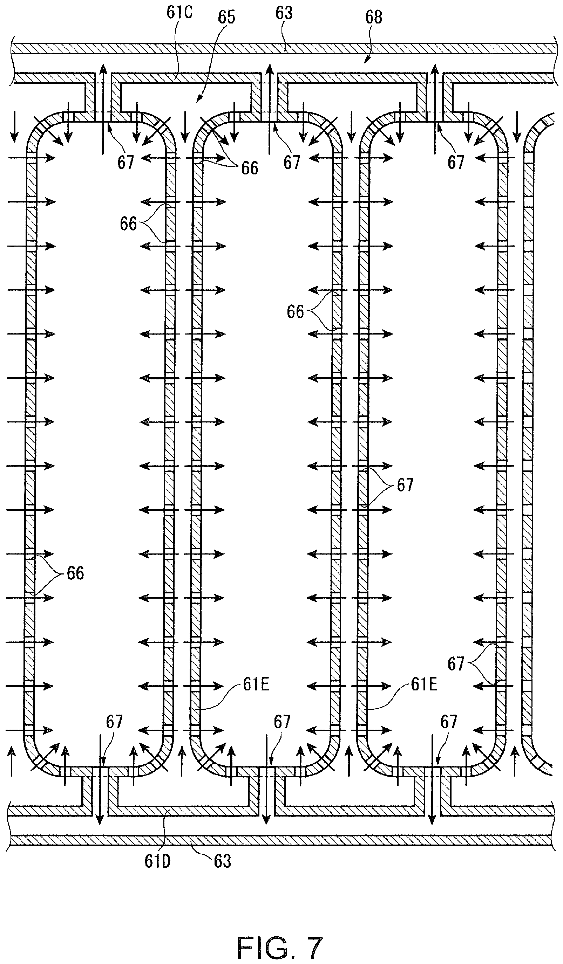

Fig. 7 is a schematic sectional view illustrating a configuration of a part of the coupler 60.

The frame body 61 (the first support portion 61A, the second support portion 61B, the third support portion 61C, the fourth support portion 61D, and the crossbeams 61E) of the coupler 60 has cavities therein as illustrated in Fig. 7, and the cavities form flow passages (supply flow passages 65) through which an acoustic matching material flows. The supply flow passages 65 communicate from the frame body 61 to the crossbeams 61E, and also communicate with the connector portion 64. In other words, the supply flow passages 65 communicate with each other between the first support portion 61A and the third support portion 61C, between the first support portion 61A and the fourth support portion 61D, between the second support portion 61B and the third support portion 61C, between the second support portion 61B and the fourth support portion 61D, between the third support portion 61C and the crossbeam 61E, and between the fourth support portion 61D and the crossbeam 61E.

The frame portion 6A of the frame body 61 is provided with ejection ports 66 at a predetermined interval over the inner peripheral surface. In other words, a plurality of ejection ports 66 are provided along the surface of the first support portion 61A on the +Y side, along the surface of the second support portion 61B along the -Y side, along the surface of the third support portion 61C along the -X side, along the surface of the fourth support portion 61D on the +X side, and the surfaces of the respective crossbeams 61E along the ±Y sides. The ejection ports 66 are also provided on the curved surfaces R, and thus air bubbles are prevented from being formed at the corners.

The ejection port 66 communicates with the supply flow passage 65, and if an acoustic matching material (gel or the like) is supplied to the supply flow passage 65, the acoustic matching material is ejected to the outside from the ejection port 66, that is, onto the acoustic lens 24 in the frame inside of each frame portion 6A. If the acoustic matching material is ejected from the ejection port 66 in a state in which the coupler 60 is attached to the ultrasonic probe 20, and the ultrasonic probe unit 2 is fixed to a living body, a space interposed among the living body, the coupler 60, and the acoustic lens 24 is filled with the acoustic matching material. At this time, as illustrated in Fig. 7, the acoustic matching material is supplied from the inner peripheral surface of each frame portion 6A toward the frame center, and, thus, the acoustic matching material can be spread uniformly and quickly to the inside of the frame portion 6A compared with, for example, a case where the acoustic matching material is supplied from a single location at an end of the acoustic lens 24.

In the present embodiment, a description has been made of an example in which the ejection ports 66 are provided on the inner peripheral surface of each frame portion 6A, but are not necessarily required to be provided on the inner peripheral surface. For example, if a direction which is orthogonal to the X direction and the Y direction is set to a Z direction (a side directed toward a living body is a +Z side, and the casing 21 side is a -Z side), in a case where a surface of the frame body 61 on the +Z side protrudes further toward the +Z side than a surface of the crossbeam 61E on the +Z side, the surface (a surface facing a living body) of the crossbeam 61E on the +Z side may also be provided with the ejection ports 66.

Meanwhile, in the present embodiment, an acoustic matching material is supplied from the inner peripheral surface of each frame portion 6A toward the frame inside. Thus, a gas present in the frame inside is required to be released out of the frame inside.

In relation to this, in the present embodiment, as illustrated in Fig. 7, discharge ports 67 are provided at each frame portion 6A. Specifically, the discharge ports 67 discharging an acoustic matching material with which a gas is mixed are provided at an intermediate position between the first support portion 61A and the crossbeam 61E located on the first support portion 61A side among the crossbeams 61E, an intermediate position between the second support portion 61B and the crossbeam 61E located on the second support portion 61B side among the crossbeams 61E, and intermediate positions between the crossbeams 61E adjacent to each other in the third support portion 61C and the fourth support portion 61D.

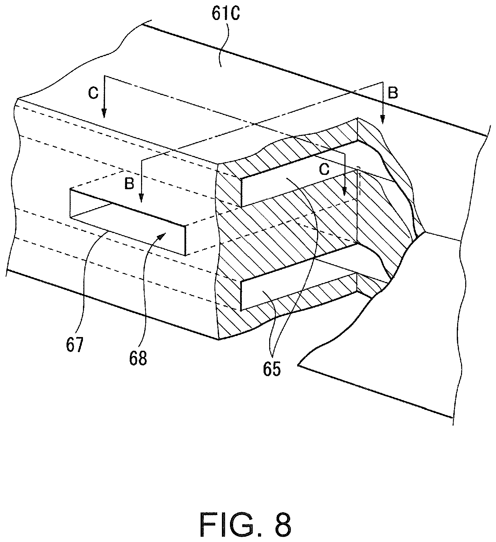

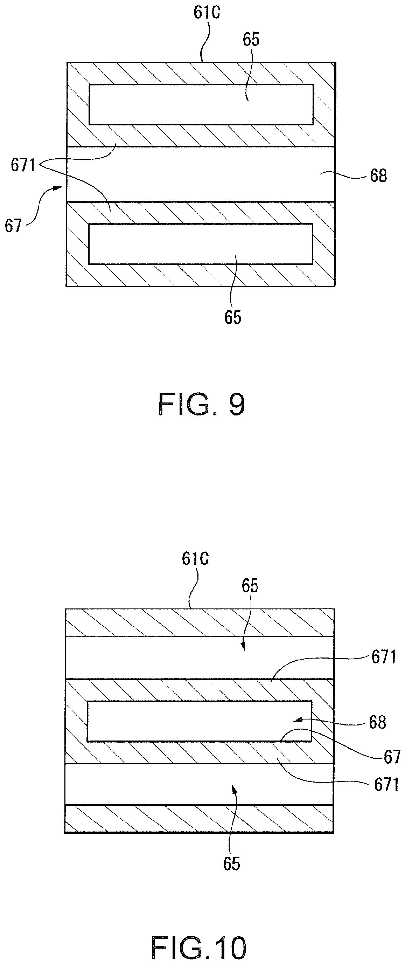

Fig. 8 is a sectional perspective view illustrating a schematic configuration of the vicinity of the discharge port 67 in the present embodiment. Fig. 9 is a sectional view taken along the line B-B in Fig. 8, and Fig. 10 is a sectional view taken along the line C-C in Fig. 8. Figs. 8 to 10 exemplify a configuration of the discharge port 67 provided at the third support portion 61C, but this is also the same for the fourth support portion 61D.

As illustrated in Figs. 8 to 10, in the third support portion 61C (fourth support portion 61D), the supply flow passage 65 along the Y direction and an deaeration passage 68 which communicates with the discharge port 67 along the X direction intersect each other at the position of the discharge port 67. For example, the deaeration passage 68 communicating with the discharge port 67 is provided at the intermediate position of the third support portion 61C in the Z direction, and the supply flow passages 65 along the X direction are provided via partition walls 671 of the deaeration passage 68 on the ±Z sides. In this configuration, even if the deaeration passage 68 is provided, an acoustic matching material can be widely spread to the supply flow passages 65 over the frame body 61 and the crossbeams 61E. In the present embodiment, a description has been made of an example in which the deaeration portion 63 is made of, for example, a plastic resin and is provided in the outer peripheral surface of the frame body 61, but may be formed of, for example, a tube.

In the present embodiment, the deaeration portion 63 is provided over the outer peripheral surface of the frame body 61, and the deaeration passage 68 is formed in the deaeration portion 63 and communicates with the above-described discharge port 67. The deaeration portion 63 is connected to the connector portion 64, and thus an acoustic matching material containing a gas discharged to the deaeration passage 68 from the connector portion 64 is discharged to the outside. Consequently, an acoustic matching material containing a gas (there are air bubbles) is discharged to the deaeration passage 68 from the discharge port 67, and thus an acoustic matching material with less air bubbles can fill an upper part of the acoustic lens 24.

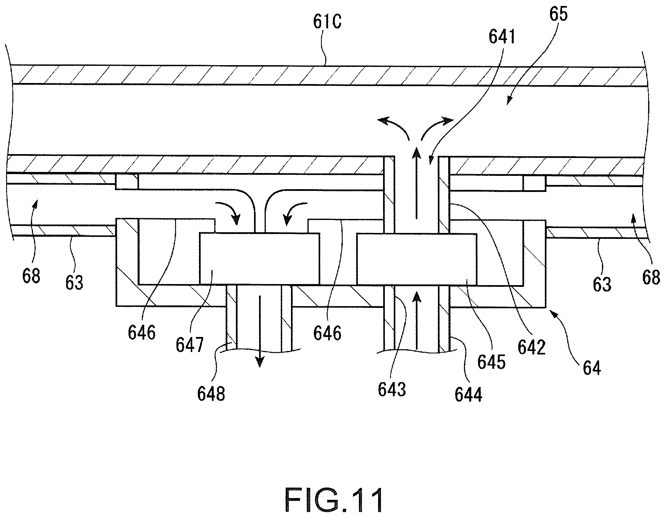

Fig. 11 is a diagram illustrating a schematic configuration of the connector portion 64 of the present embodiment.

As illustrated in Fig. 2 or 6, the connector portion 64 is provided on a part of the outer peripheral surface of the frame body 61, for example, on the outer peripheral surface of the third support portion 61C, and controls a supplying amount of an acoustic matching material and a discharge amount of an acoustic matching material.

Specifically, an opening 641 communicating with the supply flow passage 65 is provided in the frame body 61 (third support portion 61C) at the position where the connector portion 64 is provided, and the connector portion 64 has an introduction pipe 642 connected to the opening 641. The connector portion 64 has an introduction port 643, and the introduction port 643 is connected to a first connection pipe 644 which is connected to the cartridge tank 5. A first electromagnetic valve 645 is provided between the introduction port 643 and the introduction pipe 642, and the first electromagnetic valve 645 is controlled by, for example, a controller 70 (or the control device 3) connected to the connector portion 64 so that an opening or closing amount thereof is controlled. Consequently, an acoustic matching material which is supplied due to pressure in the cartridge tank 5 is supplied from the connector portion 64 into the supply flow passage 65, and a supply amount thereof is controlled.

The connector portion 64 is connected to the deaeration portion 63. The connector portion 64 is provided with a deaeration pipe 646 connected to the deaeration passage 68 of the deaeration portion 63. The deaeration pipe 646 is connected to a second connection pipe 648 which is connected to cartridge tank 5, via a second electromagnetic valve 647. The second electromagnetic valve 647 is opened and closed under the control of the controller 70 (or the control device 3) so as to discharge an acoustic matching material. The discharged acoustic matching material may be collected in an exhaust portion of the cartridge tank, so as to be subject to exhaust, and may be reused after being subject to, for example, a sterilization process. In a case where the same target object is measured, the acoustic matching material may be supplied to the first connection pipe 644 so as to be circulated.

In the present embodiment, a description has been made of an example in which the second electromagnetic valve 647 is provided, but any other configuration may be used, and, for example, a check valve may be provided.

The controller 70 (refer to Fig. 1) is connected to the connector portion 64 of the coupler 60, and includes a plurality of input operation units (not illustrated) controlling an opening or closing amount of the first electromagnetic valve 645 or the second electromagnetic valve 647 of the connector portion 64.

The controller 70 is communicably connected to the control device 3. If a control signal based on an ultrasonic measurement result is input from the control device 3, the controller 70 controls an opening or closing amount of the first electromagnetic valve 645 or the second electromagnetic valve 647 on the basis of the control signal.

### Control Device

Next, a description will be made of the control device 3 of the ultrasonic image display 1.

The control device 3 is formed of, for example, a calculation unit formed of a central processing unit (CPU) and the like, and a storage unit formed of a memory and the like.

The storage unit stores various programs or various data for performing ultrasonic measurement using the ultrasonic probe 20, and generation and display of an internal tomographic image of a living body based on an ultrasonic measurement result.

The calculation unit reads the various programs stored in the storage unit and executes the programs, so as to function as a transmission control portion 31, a reception control portion 32, an image forming portion 33, a display control portion 34, a coupler control portion 35, and the like, as illustrated in Fig. 1. The control device 3 may be provided with an input operation unit formed of a keyboard and the like.

The transmission control portion 31 controls the ultrasonic probe 20 to transmit an ultrasonic wave from each transmission/reception column 22B of the ultrasonic device 22.

The reception control portion 32 controls the ultrasonic probe 20 to acquire a received signal from each transmission/reception column 22B of the ultrasonic device 22.

The image forming portion 33 generates an internal tomographic image at each position of a living body X on the basis of the received signal (image signal) transmitted from the ultrasonic probe 20.

The display control portion 34 displays each generated internal tomographic image on the display device 4.

The coupler control portion 35 is an opening/closing control portion which controls opening or closing of the first electromagnetic valve 645 or the second electromagnetic valve 647, determines whether or not a sufficient acoustic matching material is supplied from the coupler 60 on the basis of a received signal, and outputs a control signal corresponding to a supply amount of the acoustic matching material to the controller 70.

### Method of Supplying Acoustic Matching Material

Next, a description will be made of a method of supplying an acoustic matching material from the coupler 60 of the ultrasonic probe unit 2 in the ultrasonic image display 1.

In the ultrasonic probe unit 2, in a case where the coupler 60 is attached to the ultrasonic probe 20, the coupler 60 comes into contact with the ultrasonic probe 20 so that the acoustic lens 24 is interposed between the +Y side surface of the first support portion 61A and the -Y side surface of the second support portion 61B in the Y direction. Consequently, the Y direction of the coupler 60 can be positioned with respect to the casing 21.

Next, the claws 621 of the attaching/detaching mechanisms 62 are engaged with the top surface 21C of the casing 21. Consequently, the X direction of the coupler 60 can be positioned. Since the crossbeams 61E have the lens facing surfaces 61E1 corresponding to the shape of the acoustic lens 24, the coupler 60 can be attached to the casing 21 without the crossbeams 61E interfering with the acoustic lens 24.

Thereafter, the ultrasonic probe unit 2 attached with the coupler 60 is fixed to a surface of the living body X.

If a measurer operates the controller 70 so as to open the first electromagnetic valve 645 and the second electromagnetic valve 647, an acoustic matching material is ejected onto the acoustic lens 24 in the frame inside from the ejection ports 66 of each frame portion 6A, and thus the acoustic matching material fills a gap between the living body and the ultrasonic probe 20 (acoustic lens 24). At this time, for example, there may be a configuration in which a preset amount of the acoustic matching material may be ejected by operating an operation button provided in the controller 70, and there may be a configuration in which the acoustic matching material is ejected for a preset time.

In this case, a measurer can easily determine whether or not the acoustic matching material is sufficient by performing an ultrasonic measurement process using the ultrasonic probe 20.

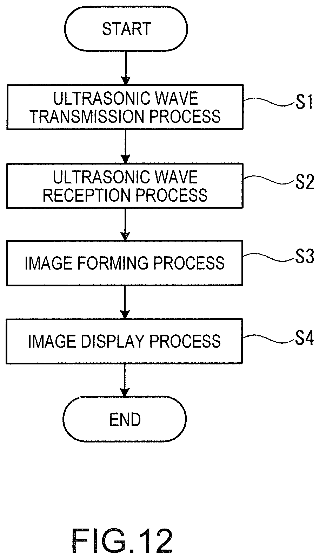

Fig. 12 is a flowchart illustrating a checking process of checking a supply amount of an acoustic matching material.

In other words, the measurer controls the control device 3 so that the transmission control portion 31 of the control device 3 controls the ultrasonic probe 20, and thus an ultrasonic wave transmission process of transmitting an ultrasonic wave from each ultrasonic transducer 22A is performed (step S1).

Next, the reception control portion 32 performs an ultrasonic wave reception process of receiving a reflected wave of the ultrasonic wave transmitted in step S1 (step S2).

Next, the image forming portion 33 forms an ultrasonic image on the basis of the received signal acquired in step S2. In other words, an image forming process of forming an internal tomographic image (ultrasonic image) of the normal living body X is performed (step S3).

Thereafter, the display control portion 34 displays the ultrasonic image formed in step S3 on the display device 4 (step S4) .

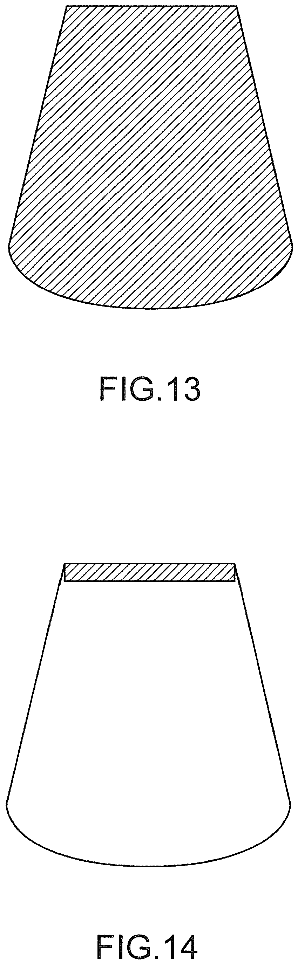

Fig. 13 illustrates an example of an ultrasonic image displayed in step S4, and illustrates an image in a case where a supply amount of an acoustic matching material is sufficient.

Fig. 14 illustrates an example of an ultrasonic image displayed in step S4, and illustrates an image in a case where a supply amount of an acoustic matching material is insufficient.

In a case where a sufficient acoustic matching material fills the gap between the ultrasonic probe 20 (acoustic lens 24) and the living body X, the ultrasonic probe 20 comes into close contact with the living body X, and thus an ultrasonic wave reflected inside the living body X is received. In this case, the ultrasonic image as illustrated in Fig. 13 is displayed.

On the other hand, in a case where a supply amount of an acoustic matching material is insufficient, the ultrasonic probe 20 does not come into close contact with the living body X, and an ultrasonic wave is reflected at an interface caused by an air bubble before reaching the living body X. In this case, a received signal becomes a strong received signal exceeding a defined value, and thus insufficiency of the acoustic matching material can be immediately detected. If an ultrasonic image is displayed in step S4, an internal tomographic image of the living body X cannot be obtained as illustrated in Fig. 14.

Therefore, the measurer visually recognizes whether or not the image as illustrated in Fig. 13 is obtained or whether or not the image as illustrated in Fig. 14 is obtained, and can thus easily determine whether or not an acoustic matching material is sufficient.

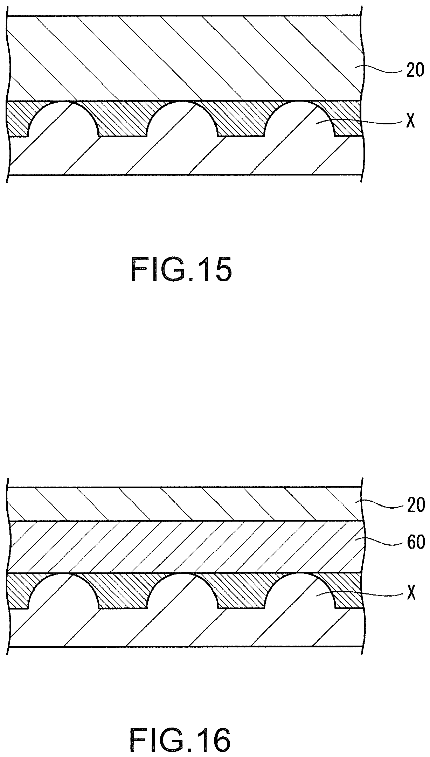

Fig. 15 is a sectional view illustrating a case where the ultrasonic probe 20 is fixed to the living body X without being attached with the coupler 60.

Fig. 16 is a sectional view illustrating a case where the ultrasonic probe 20 is fixed to the living body X in a state of being attached with the coupler 60.

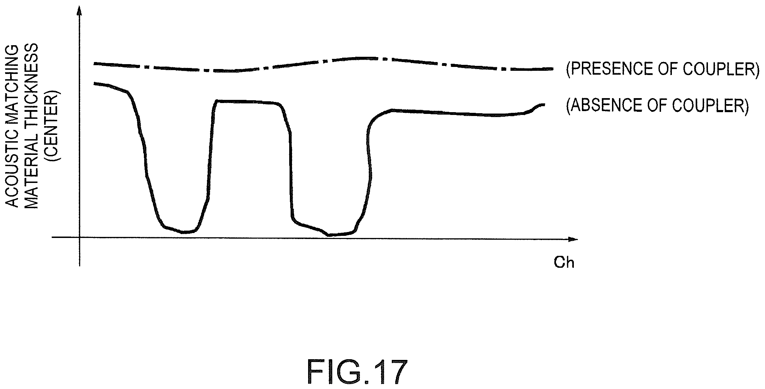

Fig. 17 is a diagram for comparing a thickness dimension of an acoustic matching material in a case where the ultrasonic probe 20 is directly fixed to the living body X as in Fig. 15 with a thickness dimension of an acoustic matching material in a case where the ultrasonic probe 20 (ultrasonic probe unit 2) attached with the coupler 60 is fixed to the living body X as in Fig. 16.

However, the living body X has a part at which a body surface is not flat due to, for example, a bone. If only the ultrasonic probe 20 is fixed to the uneven part, it is hard to bring the ultrasonic probe 20 close contact with the living body X. Therefore, as illustrated in Fig. 15 or 17, a thickness dimension of an acoustic matching material differs depending on a position at which the ultrasonic probe 20 is fixed.

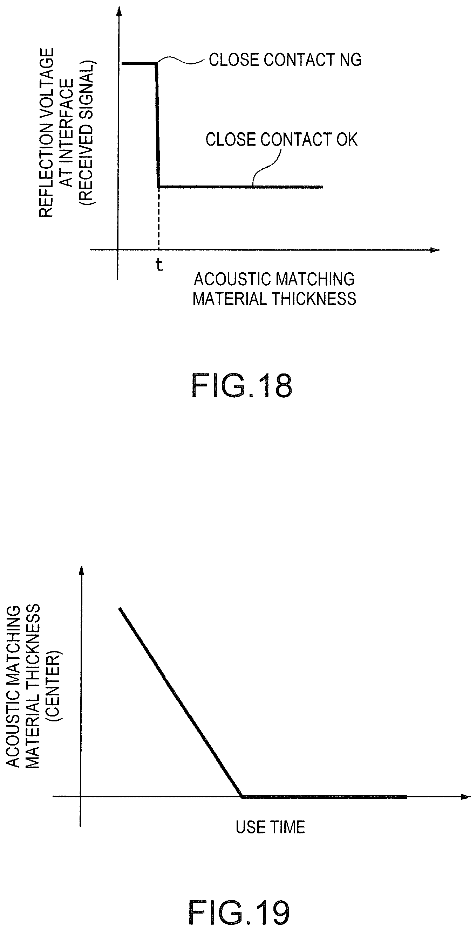

Fig. 18 is a diagram illustrating a relationship between a thickness dimension of an acoustic matching material and the intensity of a received signal based on a reflected ultrasonic wave at an interface position.

As mentioned above, if a thickness dimension of an acoustic matching material is reduced since close contact between the ultrasonic probe 20 and the living body X is reduced, as illustrated in Fig. 18, when ultrasonic wave transmission and reception processes are performed, an ultrasonic wave is reflected at an interface caused by an air bubble before reaching the living body X, and thus an accurate ultrasonic measurement process cannot be performed. In other words, in order to maintain close contact between the ultrasonic probe 20 and the living body X, a thickness dimension of an acoustic matching material of a predetermined value t or greater is necessary.

In relation to this, in the present embodiment, if the coupler 60 is attached to the ultrasonic probe 20, even in a case where the living body X is uneven, as illustrated in Figs. 16 and 17, a thickness dimension of an acoustic matching material corresponding to a thickness dimension of the coupler 60 is secured between the ultrasonic probe 20 and the living body X. Consequently, a thickness dimension of an acoustic matching material of the predetermined value t or greater is secured in Fig. 18. Therefore, the living body X and the ultrasonic probe 20 can be brought into close contact with each other, and thus it is possible to prevent the problem that ultrasonic measurement accuracy is reduced due to a reflected ultrasonic wave before reaching the living body X.

Fig. 19 is a diagram illustrating a reduction amount of an acoustic matching material over time.

As illustrated in Fig. 19, if the ultrasonic probe unit 2 is used for a long period of time, an amount of an acoustic matching material between the ultrasonic probe 20 and the living body X is reduced due to drying or the like.

Also in this case, as can be seen from Fig. 18, since a thickness dimension of an acoustic matching material is reduced, close contact between the ultrasonic probe 20 and the living body X is reduced, and thus appropriate ultrasonic measurement cannot be performed.

In the present embodiment, also in this case, an acoustic matching material can be supplied without the measurer performing work of pasting the ultrasonic probe unit 2 again according to the above-described method.

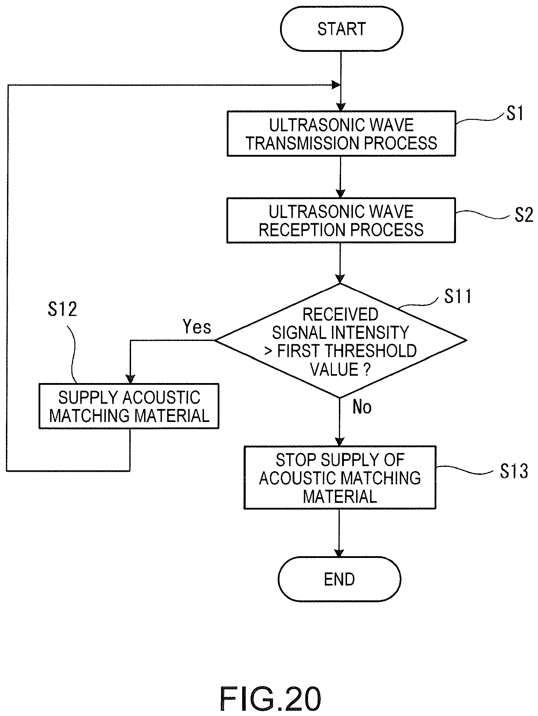

On the other hand, the above description relates to an example in which the measurer operates the controller 70 and thus an acoustic matching material is manually supplied, but an acoustic matching material may be supplied under the control of the control device 3 in the present embodiment. Fig. 20 is a flowchart illustrating a method of supplying an acoustic matching material.

In this method, after the ultrasonic probe unit 2 is fixed to the living body X, as illustrated in Fig. 20, ultrasonic wave transmission and reception processes are performed as in steps S1 and S2 in Fig. 12. Next, the coupler control portion 35 acquires the intensity of the received signal, and determines whether or not the signal intensity exceeds a first threshold value (step S11). The first threshold value corresponds to the intensity of a received signal measured in a case where an ultrasonic wave is reflected at an air bubble or the like before being incident to the living body X, and is a defined value which is sufficiently more than the intensity of a received signal during normal ultrasonic measurement.

In a case where Yes is determined in step S11, the coupler control portion 35 transmits a control signal to the controller 70 so as to open the first electromagnetic valve 645 and the second electromagnetic valve 647, and thus a predetermined amount of the acoustic matching material is supplied (step S12). Next, the flow returns to step S1.

In a case where No is determined in step S11, since the acoustic matching material is sufficient, the coupler control portion 35 transmits a control signal to the controller 70 so as to close the first electromagnetic valve 645 and the second electromagnetic valve 647 so that the supply of the acoustic matching material is stopped (step S13), and thus outflow of the acoustic matching material is prevented.

The process of supplying an acoustic matching material under the control of the control device 3 is also effective in a case where the ultrasonic probe unit 2 is fixed to the living body X and during ultrasonic measurement. In other words, during ultrasonic measurement, received signals are sequentially acquired through an ultrasonic measurement process, and thus the coupler control portion 35 can automatically supply an acoustic matching material by performing the determination in step S11 whenever the received signal is acquired.

### Operations and Effects of First Embodiment

In the present embodiment, the coupler 60 which is attachably and detachably attached to the ultrasonic probe 20 has the frame portion 6A. The frame portion 6A is provided with the supply flow passage 65 therein, and an acoustic matching material introduced from the introduction port 643 is introduced into the supply flow passage 65 and is then supplied onto the acoustic lens 24 from the plurality of ejection ports 66 provided along the inner peripheral surface of the frame portion 6A.

Thus, the acoustic matching material is supplied from an outer peripheral side toward the center with respect to a predetermined region (a region corresponding to a single transmission/reception column 22B) of the acoustic lens 24. Consequently, for example, compared with a case where an acoustic matching material is supplied from one point provided on a lateral side of the acoustic lens 24, the acoustic matching material can be spread uniformly in the predetermined region, and a supply speed increases, and thus time required to transition to an ultrasonic measurement process can be reduced.

Consequently, since a sufficient amount of an acoustic matching material can be supplied between the ultrasonic probe 20 and a living body when ultrasonic measurement using the ultrasonic probe 20 is performed, it is possible to improve close contact between the ultrasonic probe 20 and the living body X and thus to output an ultrasonic measurement result. Therefore, in the ultrasonic measurement apparatus, it is possible to perform a highly accurate ultrasonic measurement process or to form a highly accurate ultrasonic image. In the ultrasonic image display 1, since the ultrasonic image formed in the above-described way is displayed, it is possible to display an accurate ultrasonic image to a measurer, and thus to improve efficiency, for example, in diagnosis for a living body or therapy through puncturing.

In the present embodiment, a plurality of frame portions 6A are configured to be connected to each other along the X direction, and the supply flow passages 65 also communicate with each other.

In this configuration, an acoustic matching material can be supplied onto the acoustic lens 24 from the inner peripheral surface of each of the plurality of frame portions 6A. Therefore, for example, compared with a case where an acoustic matching material is supplied from only the ejection ports 66 provided at the frame body 61, an acoustic matching material can be uniformly supplied to the entire acoustic lens 24, and the acoustic matching material can be made to rapidly fill a gap between the ultrasonic probe 20 and the living body X.

In the present embodiment, a plurality of crossbeams 61E are crosslinked between the third support portion 61C and the fourth support portion 61D, and thus the rectangular frame portions 6A along the X direction (the column direction of the transmission/reception column 22B) are disposed along the Y direction. In this configuration, an acoustic matching material can be uniformly supplied to a position overlapping each transmission/reception column 22B from each crossbeam 61E.

In the present embodiment, there is provided the attaching/detaching mechanism 62 which positions the coupler 60 with respect to the ultrasonic probe 20 so that each of the plurality of crossbeams 61E is located between the transmission/reception columns 22B adjacent to each other. In other words, the Y direction is positioned by bringing the inner peripheral surfaces of the first support portion 61A and the second support portion 61B into contact with end surfaces of the acoustic lens 24 on the ±Y sides, and the X direction is positioned by engaging the claws 621 of the coupler 60 provided on the ±X sides of the frame body 61 with the casing 21, so that the coupler 60 is fixed to the casing 21. Thus, as described above, the coupler 60 can be attached and detached so that the crossbeams 61E avoid the transmission/reception columns 22B. Consequently, ultrasonic wave transmission and reception processes in each transmission/reception column 22B are not hindered by the crossbeams 61E, and thus it is possible to perform appropriate ultrasonic measurement.

In the present embodiment, the discharge port 67 discharging a gas contained in an acoustic matching material and the deaeration passage 68 communicating with the discharge port 67 are provided at the third support portion 61C and the fourth support portion 61D of the coupler 60.

Thus, even in a case where an acoustic matching material is supplied to a closed space among the living body X, the coupler 60, and the ultrasonic probe 20 (acoustic lens 24), an internal gas can be released to the deaeration passage 68, and thus it is possible to prevent air bubbles from entering a gap between the ultrasonic probe 20 and the living body X. Therefore, it is possible to improve ultrasonic measurement accuracy.

In the present embodiment, the first electromagnetic valve 645 is provided at the connector portion 64 so as to be opened and closed, and thus an amount of an acoustic matching material introduced into the supply flow passage 65 is controlled. Consequently, it is possible to control the supply of an acoustic matching material with a simple configuration.

In the present embodiment, the coupler control portion 35 of the control device 3 controls the supply of an acoustic matching material on the basis of the intensity of a received signal in ultrasonic measurement using the ultrasonic probe unit 2. Consequently, even when an ultrasonic measurement process is performed, it is possible to perform a process of supplying the acoustic matching material on the basis of the intensity of a measured received signal, and thus to prevent a problem in which measurement accuracy deteriorates due to a reduction in the acoustic matching material during ultrasonic measurement.

### Second Embodiment

Next, a second embodiment will be described.

In the first embodiment, a description has been made of an example in which all of the supply flow passages 65 of the frame body 61 and the plurality of crossbeams 61E are connected to each other, that is, the supply flow passages 65 of the respective frame portions 6A arranged in the Y direction communicate with each other. In contrast, the second embodiment is different from the first embodiment in that supply flow passages are separate from each other in the plurality of frame portions 6A arranged in the Y direction. In the following description, a constituent element which has already been described is given the same reference numeral, and a description thereof will be omitted or made briefly.

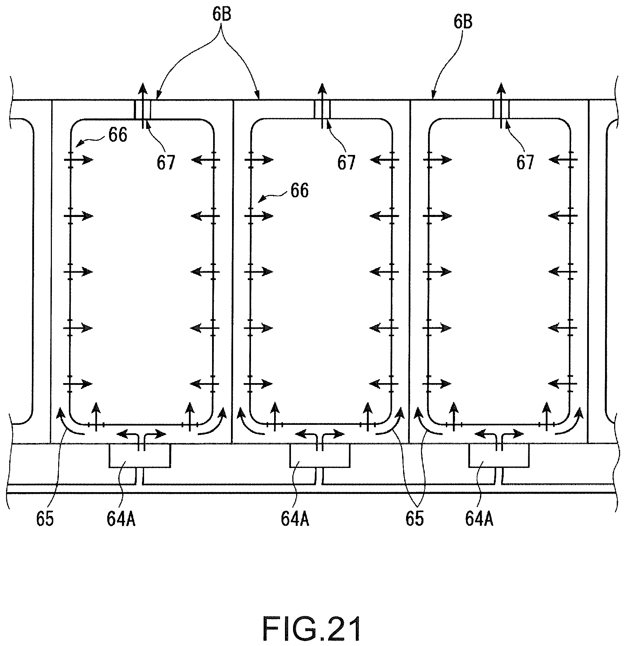

Fig. 21 is a diagram illustrating a configuration of supply flow passages of a coupler 60A according to the second embodiment.

In the present embodiment, as illustrated in Fig. 21, a plurality of frame portions 6B are arranged separately from each other along the Y direction and are connected to each other. On the other hand, each frame portion 6B has a cavity therein, and the cavity forms the same supply flow passage 65 as in the first embodiment, but the supply flow passages 65 of the frame portions 6B adjacent to each other do not communicate with each other, and are separate from each other. Each frame portion 6B is provided with a plurality of ejection ports 66 on an inner peripheral surface intersecting the measurement surface 21A in the same manner as in the first embodiment, and an acoustic matching material from the supply flow passage 65 is ejected onto the acoustic lens 24 in a frame inside region of the frame portion 6B from the ejection ports 66.

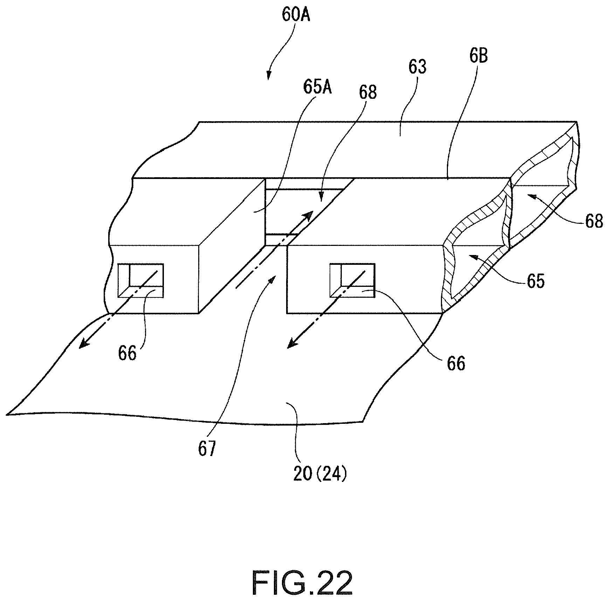

Fig. 22 is a perspective view illustrating a configuration of the vicinity of the discharge port 67 of the present embodiment.

In the present embodiment, as illustrated in Fig. 21, a single discharge port 67 is provided on an opposite side to a connector portion 64A. Configurations of the discharge port 67 and the deaeration passage 68 (refer to Fig. 22) may be the same as the configurations in the first embodiment, but the discharge port 67 may be formed by notching a part of the frame portion 6B. In this case, an acoustic matching material can be prevented from outflowing to the discharge port 67 by providing a wall 65A in the supply flow passage 65.

The discharge port 67 is formed by opening a surface on the side coming into contact with the living body X, but the surface of the discharge port 67 on the +Z side is closed by the living body X when coming into contact with the living body X, and thus an acoustic matching material containing air bubbles flows into the deaeration passage 68.

In this configuration, it is not necessary to cause the supply flow passages 65 with the discharge port 67 interposed therebetween to communicate with each other, and thus an opening area of the discharge port 67 can be increased compared with the first embodiment, so that air bubbles can be effectively released to the deaeration passage 68.

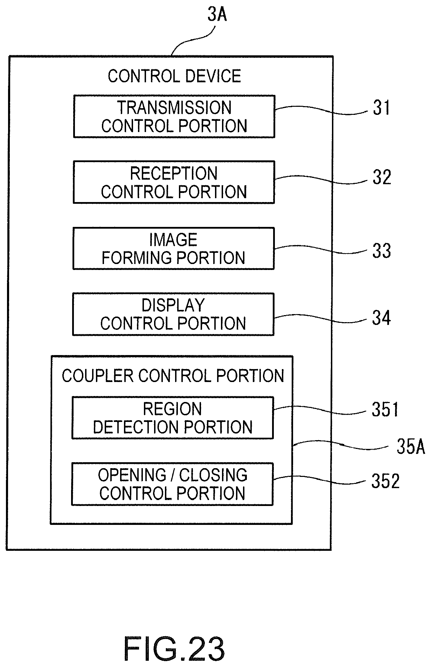

Fig. 23 is a diagram illustrating a functional configuration of a control device 3A according to the present embodiment.

The control device 3A of the present embodiment functions as transmission control portion 31, a reception control portion 32, an image forming portion 33, a display control portion 34, and a coupler control portion 35A, and the coupler control portion 35A functions as a region detection portion 351 and an opening/closing control portion 352 as illustrated in Fig. 23.

Specifically, the region detection portion 351 determines whether or not the intensity of a received signal output from each transmission/reception column 22B is equal to or more than a first threshold value, and thus detects an insufficiency region in which an acoustic matching material is insufficient and a sufficiency region in which the acoustic matching material is sufficient.

The opening/closing control portion 352 opens the first electromagnetic valve 645 provided at the connector portion 64A of the frame portion 6B corresponding to the insufficiency region, and closes the first electromagnetic valve 645 provided at the connector portion 64A of the frame portion 6B corresponding to the sufficiency region.

Next, a description will be made of a process of supplying an acoustic matching material of the present embodiment.

In the present embodiment, since a supply amount of an acoustic matching material for each frame portion 6B can be controlled separately, the acoustic matching material can be supplied to only an insufficiency region in which the acoustic matching material is insufficient between the acoustic lens 24 and the living body X.

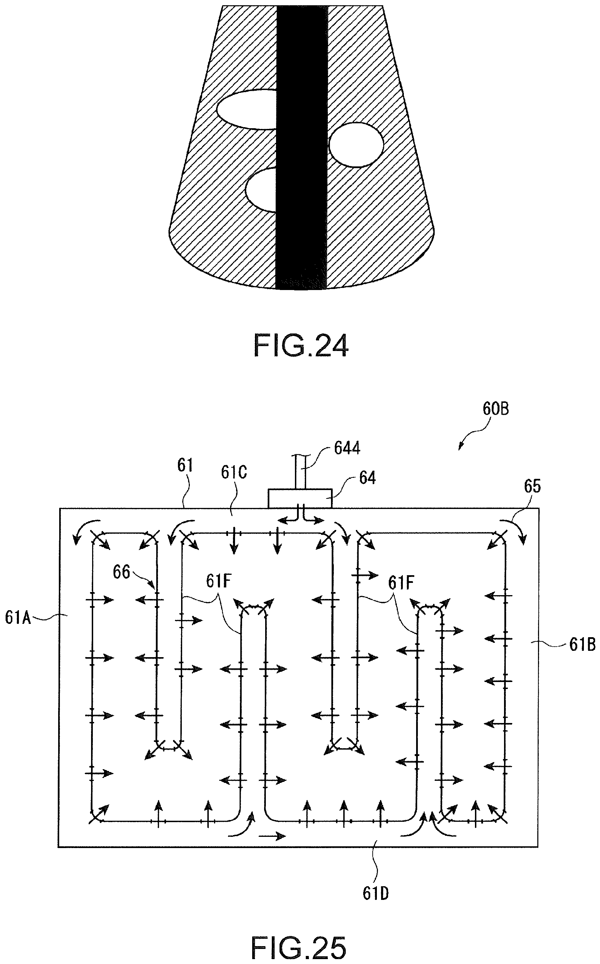

For example, in a case where a measurer manually supplies an acoustic matching material, for example, an ultrasonic image as illustrated in Fig. 24 is displayed in step S4 in Fig. 12.

Fig. 24 is a diagram illustrating an example of an ultrasonic image in a case where an acoustic matching material is insufficient in a partial region on the acoustic lens 24. In a case where an acoustic matching material is insufficient in a partial region on the acoustic lens 24, a signal whose intensity is equal to or more than a defined value is received in the transmission/reception column 22B corresponding to the region.

Thus, a portion corresponding to the region is not displayed as illustrated in Fig. 24.

In this case, a measurer operates the controller 70 so as to open the first electromagnetic valve 645 provided at the connector portion 64A of the frame portion 6B corresponding to the region whose image is not displayed. Consequently, an acoustic matching material is supplied to only the region in which the acoustic matching material is insufficient.

On the other hand, this is also the same for a case where an acoustic matching material is automatically supplied by the control device 3A as illustrated in Fig. 20.

In other words, in step S11 in Fig. 20, the region detection portion 351 of the coupler control portion 35A detects a received signal from each transmission/reception column 22B, and determines whether or not the intensity of the received signal exceeds the first threshold value. As described above, an insufficiency region is determined with respect to the transmission/reception column 22B which outputs a received signal whose intensity exceeds the first threshold value, and a sufficiency region is determined with respect to the transmission/reception column 22B which outputs a received signal whose intensity is equal to or less than the first threshold value.

In step S12, the opening/closing control portion 352 opens the first electromagnetic valve 645 provided at the connector portion 64A of the frame portion 6B corresponding to the insufficiency region, and closes the first electromagnetic valve 645 provided at the connector portion 64A of the frame portion 6B corresponding to the sufficiency region. Consequently, in step S12, it is possible to separately perform a process of supplying an acoustic matching material to each frame portion 6B.

### Operations and Effects of Second Embodiment

In the second embodiment, the supply flow passages 65 of the respective frame portions 6B do not communicate with each other, and the connector portion 64A is provided in each frame portion 6B. The region detection portion 351 determines an insufficiency region and a sufficiency region on the basis of a received signal from each transmission/reception column 22B, and the opening/closing control portion supplies an acoustic matching material to the insufficiency region, and stops the supply of the acoustic matching material to the sufficiency region.

In this case, it is possible to supply an acoustic matching material to only the insufficiency region without excessively supplying the acoustic matching material to the sufficiency region on the acoustic lens 24. Consequently, it is possible to uniformly supply the acoustic matching material on the acoustic lens 24. The excessive supply of the acoustic matching material is prevented, and thus it is possible to reduce cost.

In the present embodiment, a notch portion formed by notching a part of the frame portion 6B is provided, and the notch portion forms the discharge port 67 and further forms the deaeration passage 68.

Thus, it is possible to discharge a gas contained in an acoustic matching material with a simple configuration, and also to efficiently discharge air bubbles since an opening area of the discharge port 67 can be increased.

### Third Embodiment

Next, a third embodiment will be described.

In the first embodiment, a description has been made of an example in which the crossbeam 61E crosslinked between the third support portion 61C and the fourth support portion 61D of the frame body 61 is provided. In contrast, in the third embodiment, there is a difference in that a protrusion is provided instead of the crossbeam 61E.

Fig. 25 is a diagram illustrating a configuration of a supply flow passage of a coupler 60B according to the third embodiment.

In the present embodiment, as illustrated in Fig. 25, the coupler 60B includes not only the substantially same frame body 61 as in the first embodiment, but also protrusions 61F protruding toward the center of the frame inside from the third support portion 61C and the fourth support portion 61D of the frame body 61.

The protrusion 61F is provided to be located between the transmission/reception columns 22B adjacent to each other when the coupler 60B is attached to the ultrasonic probe 20 in the same manner as the crossbeam 61E.

A surface (a surface facing a living body) of the protrusion 61F on the +Z side is formed in a curved surface corresponding to a surface shape of the acoustic lens 24 in the same manner as in the crossbeam 61E.

The protrusion 61F has the cavity therein, and the cavity communicates with the supply flow passage 65 of the third support portion 61C or the fourth support portion 61D so as to form a part (branching passage) of the supply flow passage 65.

A plurality of ejection ports 66 (second ejection ports) are provided on the side surface of the protrusion 61F intersecting the measurement surface 21A, and an acoustic matching material flowing through the supply flow passage 65 is ejected onto the acoustic lens 24.

### Operations and Effects of Third Embodiment

In the present embodiment, the protrusions 61F provided with the supply flow passages 65 protrude toward the frame inside from the third support portion 61C and the fourth support portion 61D of the frame body 61, and a plurality of ejection ports 66 are provided on the side surfaces of the protrusions 61F. Thus, in the same manner as in the first embodiment, compared with a case where an acoustic matching material is supplied from a single location in an outer peripheral portion of the acoustic lens 24, an acoustic matching material can be widely and efficiently spread on the acoustic lens 24, and thus the acoustic matching material can be uniformly supplied.

### Fourth Embodiment

Next, a fourth embodiment will be described with reference to the drawings.

In the first embodiment, an acoustic matching material is supplied from an outer peripheral side toward the center with respect to a predetermined region (a region of the frame inside of the frame portion 6A) on the acoustic lens 24. In contrast, in the present embodiment, there is a difference in that an acoustic matching material is supplied from the center toward the outer peripheral side in the region of the frame inside of the frame portion 6A.

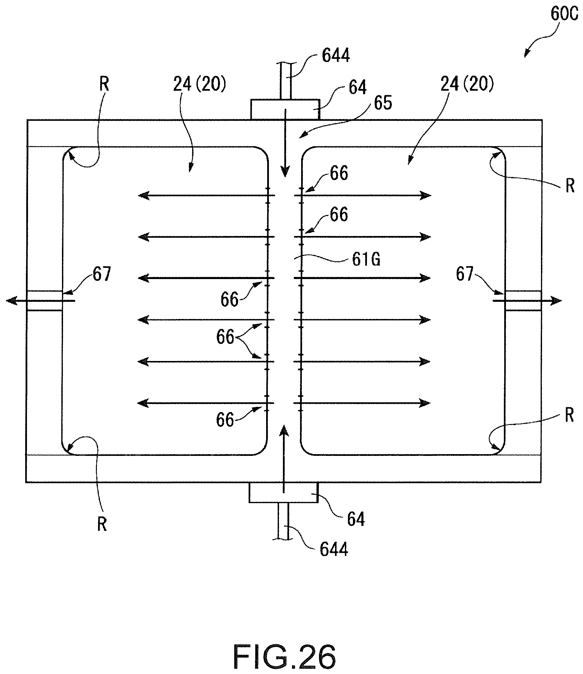

Fig. 26 is a diagram illustrating a configuration of a supply flow passage of a coupler 60C according to the fourth embodiment.

In the present embodiment, as illustrated in Fig. 26, in the substantially same manner as in the first embodiment, the coupler 60C is provided with a frame body 61 including a first support portion 61A, a second support portion 61B, a third support portion 61C, and a fourth support portion 61D, and the frame body 61 forms a single frame portion. The coupler 60C includes a central extending portion 61G which extends toward the center of the frame body 61 from the third support portion 61C in a central portion of the frame body 61 in the Y direction and is connected to the fourth support portion 61D. In the present embodiment, the first support portion 61A, the second support portion 61B, the third support portion 61C, and the fourth support portion 61D are not provided with the ejection ports 66.

The frame body 61 is a flow passage forming portion, and is connected to the connector portion 64 at the third support portion 61C and the fourth support portion 61D, and the supply flow passage 65 is formed inside the third support portion 61C and the fourth support portion 61D.

The inside of the central extending portion 61G is also a cavity, and is connected to the supply flow passage 65 of the third support portion 61C or the fourth support portion 61D. In other words, the supply flow passage 65 is also formed in the central extending portion 61G.

In the central extending portion 61G, a plurality of ejection ports 66 (central ejection ports) are provided on side surfaces intersecting the measurement surface 21A of the casing 21 and directed toward ±Y sides, and, as indicated by arrows in Fig. 26, an acoustic matching material is ejected from the center of the frame body 61 outward (±Y sides).

In this case, the discharge port 67 is preferably provided at, for example, the first support portion 61A or the second support portion 61B located on a downstream side in a direction in which an acoustic matching material is supplied.

### Operations and Effects of Fourth Embodiment

In the present embodiment, an acoustic matching material is ejected toward the center in a region surrounded by the frame body 61 from the ejection ports 66 provided at the central extending portion 61G, and is thus supplied from the center outward. Also in this configuration, for example, compared with a case where an acoustic matching material is supplied from a single point in an outer peripheral portion of the acoustic lens 24, the acoustic matching material can be efficiently supplied in a wide range centering on the region center.

### Fifth Embodiment

Next, a fifth embodiment will be described with reference to the drawings.

In the above-described respective embodiments, the fixation type ultrasonic probe 20 fixed to the living body X has been exemplified. In contrast, the fifth embodiment shows an example in which the acoustic matching material supply device is applied to an ultrasonic probe 20A which is used in the related art and in which a measurer holds the ultrasonic probe with the hands thereof so as to operate an attitude of the ultrasonic probe or transmission and reception directions of ultrasonic waves.

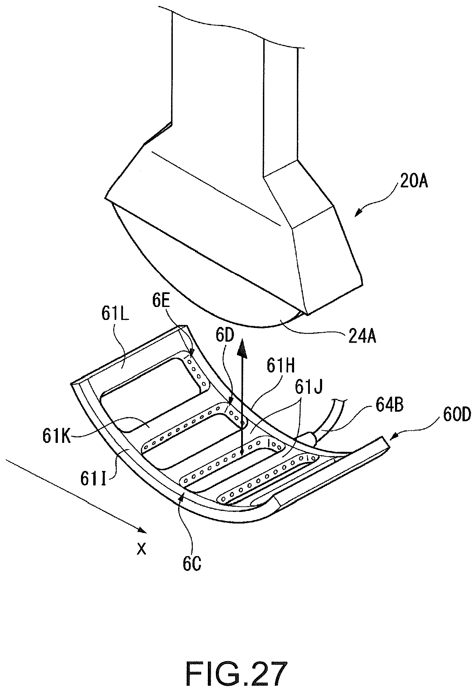

Fig. 27 is a diagram illustrating schematic configurations of the ultrasonic probe 20A (convex type probe) used in the related art and a coupler 60D which is attachably and detachably attached to the ultrasonic probe 20A.

In the convex type ultrasonic probe 20A, generally, a bulk type piezoelectric body (not illustrated) is used as an ultrasonic vibrator stored therein, and the ultrasonic probe has a one-dimensional array structure in which piezoelectric bodies which are rectangular in the Y direction are disposed along a circular arc in the X direction. The ultrasonic probe 20A is provided with an acoustic lens 24A in order to cause a transmitted ultrasonic wave to converge at a focal point position at a predetermined depth. The acoustic lens 24A has a cylindrical shape as illustrated in Fig. 27, and a section thereof along a slice direction (X direction) has a substantially circular arc shape.

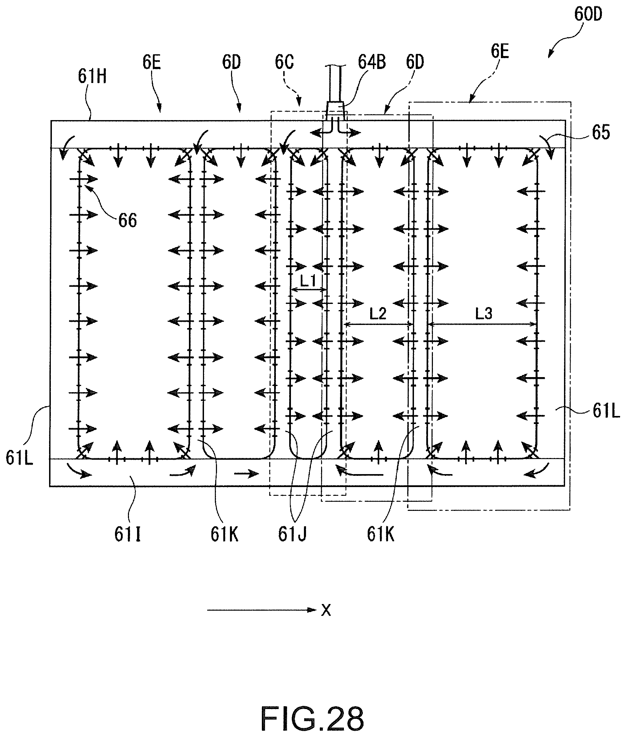

Fig. 28 is a plan view of the coupler 60D of the present embodiment. In Figs. 27 and 28, an attaching/detaching mechanism is not illustrated, but, actually, an attaching/detaching mechanism attaching the coupler 60D to the ultrasonic probe 20A is provided. The attaching/detaching mechanism may exemplify a claw as in the first embodiment.

As illustrated in Fig. 28, the coupler 60D of the present embodiment is provided with a pair of a fifth support portion 61H and a sixth support portion 61I along the X direction, and a plurality of crosslinking portions 61J, 61K and 61L crosslinked between the fifth support portion 61H and the sixth support portion 61I.

Here, as illustrated in Fig. 28, a pair of crosslinking portions 61J are disposed at the center of the coupler 60D in the X direction with a gap dimension L1. A pair of crosslinking portions 61K are respectively disposed with a distance L2 near the crosslinking portions 61J. A pair of crosslinking portions 61L are disposed with a distance L3 near the crosslinking portions 61K. The distances L1, L2 and L3 satisfy a relationship of L1<L2<L3.

In the present embodiment, a central frame portion 6C (first frame portion) is formed of the pair of crosslinking portions 61J, the fifth support portion 61H, and the sixth support portion 61I. An intermediate frame portion 6D is formed of the crosslinking portion 61J, the crosslinking portion 61K adjacent to the crosslinking portion 61J, the fifth support portion 61H, and the sixth support portion 61I. An end frame portion 6E (second frame portion) is formed of the crosslinking portion 61K, the crosslinking portion 61L adjacent to the crosslinking portion 61K, the fifth support portion 61H, and the sixth support portion 61I.

The insides of the fifth support portion 61H, the sixth support portion 61I, and the crosslinking portions 61J, 61K and 61L are cavities, and the cavities are connected to each other so as to form the supply flow passage 65 of the acoustic matching material. In the same manner as in the first embodiment, a plurality of ejection ports 66 are provided on each side surface (frame inner peripheral surface) intersecting the surface of the acoustic lens 24A when the coupler 60D is attached to the ultrasonic probe 20A in the fifth support portion 61H, the sixth support portion 61I, and crosslinking portions 61J, 61K and 61L. The connector portion 64B is provided at the fifth support portion 61H, and an acoustic matching material is introduced into the supply flow passage from an introduction port (not illustrated).

Therefore, also in the present embodiment, in the same manner as in the first embodiment, an acoustic matching material can be supplied to the center from an outer peripheral side on the acoustic lens 24A on a frame inner peripheral side of each frame portion (the central frame portion 6C, the intermediate frame portions 6D, and the end frame portions 6E) .

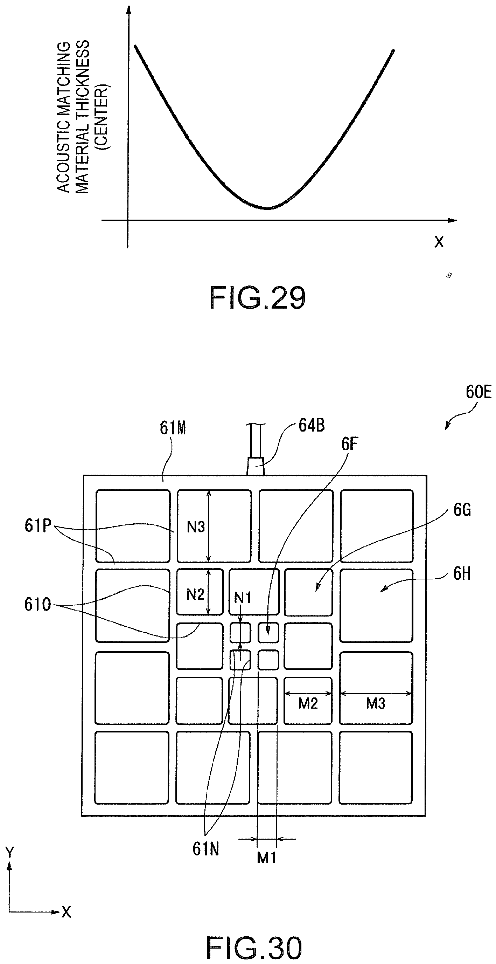

Fig. 29 is a diagram illustrating a thickness dimension of an acoustic matching material in a case where the ultrasonic probe 20A is used.

Meanwhile, in a case where the ultrasonic probe 20A is used, since the acoustic lens 24A has a circular arc shape in which the center considerably projects, if a measurer tightly presses the ultrasonic probe 20A against a living body so as to perform ultrasonic measurement, as illustrated in Fig. 29, particularly, an acoustic matching material is dried or is pushed to other positions in the vicinity of the central projection (the midpoint in the X direction) of the acoustic lens 24A, so that a thickness dimension of the acoustic matching material is reduced.

Therefore, close contact with the living body X is reduced in the central projection of the ultrasonic probe 20A for which an ultrasonic measurement result is most necessary, and thus the ultrasonic measurement accuracy deteriorates due to mixing of air bubbles or the like.

In contrast, in the present embodiment, as described above, the relationship of L1<L2<L3 is satisfied. In other words, a volume of the frame inside of the central frame portion 6C located at the center in the X direction is small, and a volume of the frame inside of the intermediate frame portion 6D located on the end side in the X direction increases. A volume of the frame inside of the end frame portion 6E is larger than the volume of the frame inside of the intermediate frame portion 6D.

Therefore, if an acoustic matching material is supplied to the supply flow passage from the connector portion 64B, the acoustic matching material fills the frame inside of the central frame portion 6C having the small volume faster than the intermediate frame portion 6D or the end frame portion 6E. It is possible to rapidly compensate for a shortage of the acoustic matching material.

### Operations and Effects of Fifth Embodiment

The coupler 60D of the present embodiment which is attachable to the convex type ultrasonic probe 20A includes the central frame portion 6C, the intermediate frame portion 6D, and the end frame portion 6E connected to each other along the X direction. The insides of the fifth support portion 61H, the sixth support portion 61I, and the crosslinking portions 61J, 61K and 61L forming the central frame portion 6C, the intermediate frame portion 6D, and the end frame portion 6E are cavities, and the cavities are connected to each other so as to form the supply flow passage. The connector portion 64B which supplies the acoustic matching material from the introduction port to the supply flow passage is connected to the fifth support portion 61H. The fifth support portion 61H, the sixth support portion 61I, and the crosslinking portions 61J, 61K and 61L have the ejection ports which eject an acoustic matching material toward the frame insides of the central frame portion 6C, the intermediate frame portion 6D, and the end frame portion 6E. Thus, in the same manner as in the first embodiment, an acoustic matching material can be supplied from an outer periphery of each frame inside region toward the center on the acoustic lens 24A, and thus the acoustic matching material can be uniformly and rapidly disposed on the acoustic lens 24A.

In the present embodiment, a width dimension (the distance L1 between the pair of crosslinking portions 61J) of the central frame portion 6C along the X direction is smaller than a width dimension of the intermediate frame portion 6D or the end frame portion 6E provided at the end in the X direction, along the X direction. Therefore, an acoustic matching material can be rapidly supplied by using the frame inside of the central frame portion 6C in which a reduction ratio of the acoustic matching material is high. Consequently, even in a case where the convex type ultrasonic probe 20A is tightly pressed against a living body, and ultrasonic measurement is performed, it is possible to rapidly perform highly accurate measurement.

### Sixth Embodiment

In the above-described fifth embodiment, a description has been made of an example in which the convex type ultrasonic probe 20A is provided with the frame portions 6C, 6D and 6E whose width dimensions increase from the center toward the end along the X direction. In contrast, in the sixth embodiment, width dimensions along the Y direction also increase from the center toward the end along the Y direction.

Fig. 30 is a plan view illustrating a schematic configuration of a coupler 60E according to the sixth embodiment.

The coupler 60E includes an outer peripheral frame 61M, crosslinking portions 61N along the X direction and the Y direction, forming a central frame portion 6F (third frame portion) disposed at the center with respect to the acoustic lens 24A, crosslinking portions 610 forming an intermediate frame portion 6G disposed on an outer peripheral side of the central frame portion 6F, and crosslinking portions 61P forming an end frame portion 6H (fourth frame portion) disposed on an outer peripheral side of the intermediate frame portion 6G.

In the same manner as in the fifth embodiment, the insides of the outer peripheral frame 61M, and the respective crosslinking portions 61N, 610 and 61P are cavities, and the cavities are connected to each other so as to form a supply flow passage.

The outer peripheral frame 61M, and the crosslinking portions 61N, 610 and 61P are provided with ejection ports from which an acoustic matching material is ejected on frame inner peripheral surfaces of the respective frame portions 6F, 6G and 6H, and the ejection ports communicate with the supply flow passage. The outer peripheral frame 61M is provided with the connector portion 64B, and an acoustic matching material is introduced into the supply flow passage.

In the present embodiment, the frame portions 6F, 6G and 6H are arranged in an array form. A width dimension M1 of the central frame portion 6F along the X direction, a width dimension N1 thereof along the Y direction, a width dimension M2 of the intermediate frame portion 6G along the X direction, a width dimension N2 thereof along the Y direction, and a width dimension M3 of the end frame portion 6H along the X direction, and a width dimension N3 thereof along the Y direction satisfy a relationship of M1<M2<M3, and a relationship of N1<N2<N3.

In other words, an area (volume) of the frame inside surrounded by the central frame portion 6F is smaller than that of the intermediate frame portion 6G or the end frame portion 6H.

In the present embodiment, the ultrasonic probe 20A is provided with ultrasonic measurement regions disposed in an array form. Specifically, bulky piezoelectric bodies are arranged in a two-dimensional array form, and the piezoelectric bodies can be driven separately.

In contrast, in the same manner as in the first embodiment, in the coupler 60E, each of the crosslinking portions 61N, 610 and 61P is disposed between the piezoelectric bodies adjacent to each other of the ultrasonic probe 20A. In other words, the crosslinking portions 61N, 610 and 61P or side surfaces thereof (frame inner peripheral surfaces of the frame portions 6F, 6G and 6H) provided with the ejection ports 66 are provided at positions not overlapping the piezoelectric bodies. Thus, in the same manner as in the first embodiment, ultrasonic wave transmission and reception processes in the crosslinking portions 61N, 610 and 61P are not hindered, and a highly accurate ultrasonic measurement process can be performed.

### Operations and Effects of Sixth Embodiment

In the sixth embodiment, in the same manner as in the fifth embodiment, for example, the central frame portion 6F having a small area (volume) is disposed at the center of the acoustic lens 24A having a curved surface, such as a convex probe, and thus it is possible to rapidly supply an acoustic matching material corresponding to a reduction amount even in a case where an amount of the acoustic matching material is reduced.

As in the present embodiment, a configuration in which the frame portions 6F, 6G and 6H are disposed in an array form is optimal for an ultrasonic device having a two-dimensional array structure. In other words, if the coupler 60E having a two-dimensional array structure is attached to an ultrasonic probe having a one-dimensional array structure as in the first embodiment, a crosslinking portion is disposed on the transmission/reception column 22B, and thus ultrasonic wave transmission and reception processes are hindered. In contrast, in a case where a plurality of ultrasonic transducers are separate from each other so as to be configured in a two-dimensional array structure, or transmission/reception portions are formed of a plurality of ultrasonic transducers, and the transmission/reception portions are disposed in a two-dimensional array structure, the coupler 60E is configured so that the ultrasonic transducers or the transmission/reception portions are disposed in the frame insides of the frame portions 6F, 6G and 6H having a two-dimensional array structure, and thus ultrasonic wave transmission and reception processes are not hindered.

### Modification Examples

Each of the above-described respective embodiments is only an example, and configurations obtained through modifications, alterations, and combinations of the embodiments within the scope of being capable of achieving the object of the invention are included in the invention.

For example, in the first embodiment, a description has been made of an example in which the coupler 60 is attachable to and detachable from the ultrasonic probe 20 so that a single frame portion 6A is disposed to correspond to each transmission/reception column 22B. In other words, a gap between the respective transmission/reception columns 22B in the coupler 60 is a gap between the transmission/reception columns 22B. In contrast, there may be a configuration in which a plurality of transmission/reception columns 22B are disposed in the frame inside of a single frame portion 6A. For example, there may be a configuration in which, when the coupler 60 is attached to the ultrasonic probe 20, two or more transmission/reception columns 22B are disposed between the crossbeams 61E adjacent to each other.

In the first embodiment, the discharge port 67 is configured to be located at an intermediate position between the crossbeams 61E adjacent to each other, that is, on an extension line of the transmission/reception column 22B, but, in a case where two or more transmission/reception columns 22B are provided between the crossbeams 61E as described above, the discharge port 67 is preferably configured to be provided between the transmission/reception columns 22B adjacent to each other. The discharge port 67 is a portion from which an acoustic matching material containing air bubbles are discharged, and is likely to collect air bubbles. Therefore, the discharge port 67 is provided at a position avoiding the transmission/reception column 22B, and thus it is possible to reduce a probability that an air bubble is present in an ultrasonic wave transmission direction or reception direction of the transmission/reception column 22B and also to improve measurement accuracy.

There is a more preferable configuration in which, when the coupler 60 is attached to the ultrasonic probe 20, an even number of transmission/reception columns 22B are disposed between the crossbeams 61E adjacent to each other, and the discharge port 67 is provided at an intermediate position (midpoint) between the crossbeams 61E adjacent to each other. In this case, since an acoustic matching material flows uniformly from the ±Y sides toward the discharge port 67, the acoustic matching material can be uniformly supplied onto the acoustic lens 24.

This is also the same for the second embodiment, and a plurality of transmission/reception columns 22B may be provided in the frame inside of each frame portion 6A.

In the first embodiment, the discharge port 67 may be formed of a notch portion obtained by notching a part of the frame body 61 (frame portion 6A) as in the second embodiment.

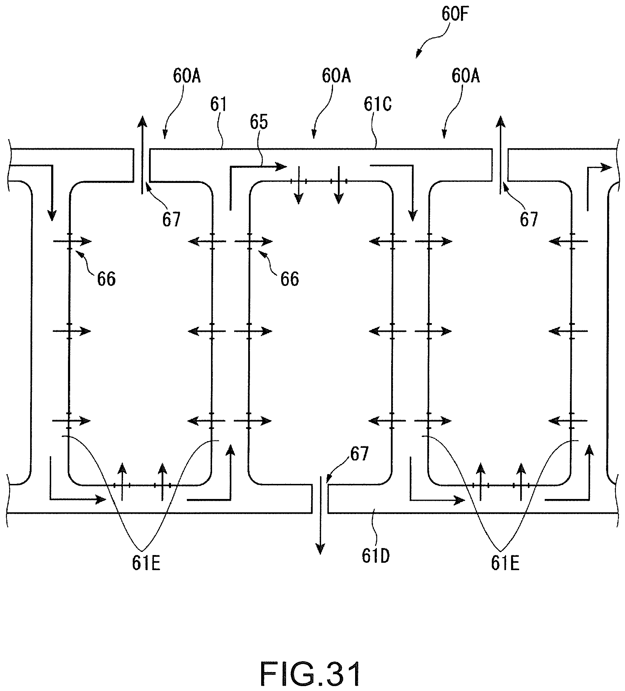

Fig. 31 is a diagram illustrating a supply flow passage according to a modification example of the first embodiment.

In this case, as in a coupler 60F illustrated in Fig. 31, a position where the discharge port 67 is provided is alternately disposed at the third support portion 61C and the fourth support portion 61D in the frame portion 6A along the X direction, and thus it is possible to prevent flow velocity of an acoustic matching material in the supply flow passage 65 from being reduced.

In the second embodiment, a plurality of frame portions 6B are configured to be connected to each other along the Y direction, but, in this case, supply flow passages 65 of two frame portions 6B are required to be formed between the transmission/reception columns 22B adjacent to each other. In this case, a diameter of the supply flow passage 65 is small, and thus a sufficient amount of an acoustic matching material may not be supplied.

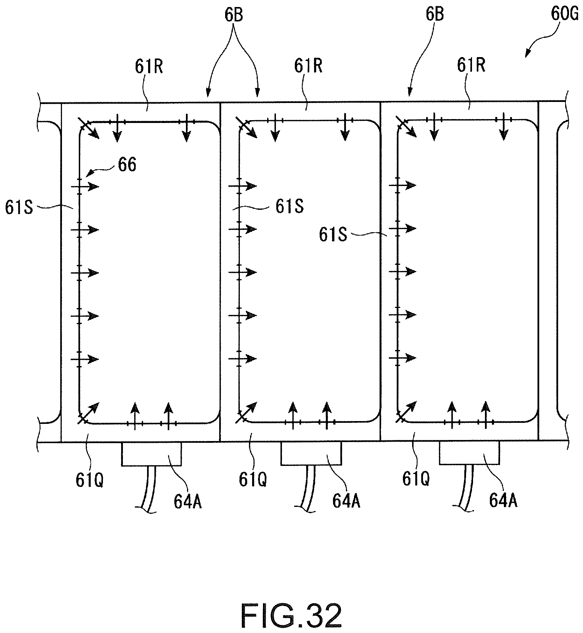

Fig. 32 is a diagram illustrating a schematic configuration of a supply flow passage according to a modification example of the second embodiment.

Compared with the second embodiment, in a coupler 60G of the modification example illustrated in Fig. 32, a supply flow passage in each frame portion 6B is formed over three sides such as a seventh support portion 61Q provided with the connector portion 64A, an eighth support portion 61R opposing the seventh support portion 61Q, and a ninth support portion 61S crosslinked between an one end of the seventh support portion 61Q and the eighth support portion 61R. In this case, the ninth support portion 61S of another frame portion 6B adjacent thereto (connected thereto) in the Y direction is in contact with and is connected to one end of the seventh support portion 61Q and the other end of the eighth support portion 61R. The supply flow passages do not communicate with each other.

In this configuration, an acoustic matching material is supplied from three directions, but a single supply flow passage may be formed between the transmission/reception columns 22B, and thus a diameter of the supply flow passage 65 is not reduced.

In the first embodiment, the frame body 61 in which a plurality of frame portions 6A are connected to each other in the Y direction has been exemplified, but is not limited thereto. For example, there may be a configuration in which the crossbeam 61E is not provided, and a single frame portion surrounded by four sides such as the first support portion 61A, the second support portion 61B, the third support portion 61C, and the fourth support portion 61D surrounds the entire acoustic lens 24.

In the fifth embodiment or the sixth embodiment, the coupler 60D or 60E attached to the convex type ultrasonic probe 20A has been exemplified, but, as in the first embodiment, in the same manner for the coupler 60 attached to a fixation type probe in which the ultrasonic probe 20 is fixed to the living body X, as a frame portion is located further toward the center, an area (volume) of a frame inside region of the frame portion may be reduced. In other words, in a frame portion corresponding to a position where a protrusion dimension of the acoustic lens 24 is large, a thickness dimension of an acoustic matching material is reduced, and the influence of a reduction in the acoustic matching material due to drying increases. In contrast, with this configuration, it is possible to rapidly supply an acoustic matching material to a region in which the influence of a reduction in the acoustic matching material is great.

In the sixth embodiment, in the ultrasonic transducer 22A, each frame portion can be positioned with respect to an ultrasonic device in which the ultrasonic transducers 22A are disposed in a two-dimensional array structure. In the ultrasonic probe 20 fixed to the living body X, in order to perform ultrasonic measurement in a wide range, there are many cases of employing a two-dimensional array structure in which the ultrasonic transducers 22A can be separately driven. Therefore, it is possible to appropriately apply the configuration of the coupler 60E to a fixation type ultrasonic probe. In this case, as in the sixth embodiment, a frame inside area (volume) of the central frame portion 6F may be smaller than that of the intermediate frame portion 6G or the end frame portion 6H, and frame inside areas of the respective frame portions may be the same as each other (width dimensions in the X direction are the same as each other, and width dimensions in the Y direction are the same as each other) .

In the second embodiment, the first electromagnetic valve 645 is controlled, and thus a supply amount of an acoustic matching material is changed in an insufficiency region and a sufficiency region. In contrast, there may be a configuration in which an opening/closing member such as a micro-shutter is provided at each ejection port 66, and the ejection portion is opened and closed by the micro-shutter. In this configuration, as in the second embodiment, it is not necessary to provide the frame portion 6B in which supply flow passages are separate from each other, and an amount of an acoustic matching material supplied to each region can also be adjusted in the coupler 60 of the first embodiment. Also in the same frame portion, a direction in which an acoustic matching material flows can be controlled by controlling the extent of opening of the ejection port. For example, the extent of opening of the ejection port 66 located near the discharge port 67 is decreased, the extent of opening of the ejection port 66 located far from the discharge port 67 is increased, and thus an unnecessary substance such as an air bubble can be made to appropriately flow toward the discharge port 67 side.

A specific structure at the time of implementing the invention may be configured by combining the respective embodiments and modification examples with each other as appropriate within the scope of being capable of achieving the object of the invention, and may be altered to other structures as appropriate.

## Claims

1. An acoustic matching material supply device comprising:
a frame portion that is attached to an ultrasonic probe including an ultrasonic sensor surface for performing at least one of transmission and reception of ultrasonic waves, and ejects an acoustic matching material,
wherein the frame portion includes
an inner peripheral surface included in a plane intersecting the ultrasonic sensor surface,
an ejection port that is provided on the inner peripheral surface or in a region including the inner peripheral surface, and ejects the acoustic matching material,
an introduction port that introduces the acoustic matching material from the outside, and
a flow passage through which the ejection port and the introduction port communicate with each other.

2. The acoustic matching material supply device according to claim 1,
wherein a plurality of the frame portions are provided to be connected to each other along the ultrasonic sensor surface.

3. The acoustic matching material supply device according to claim 2,
wherein the plurality of frame portions are connected to each other in a line along a first direction.

4. The acoustic matching material supply device according to any one of the preceding claims,
wherein the ultrasonic sensor surface has an ultrasonic measurement region, and
wherein the frame portion includes an attaching/detaching mechanism that allows the frame portion to be attached to or detached from the ultrasonic probe so that the inner peripheral surface avoids the ultrasonic measurement region.

5. The acoustic matching material supply device according to claim 2 or 3,
wherein, among the plurality of frame portions, a frame portion located at the center in the first direction has a smaller width dimension along the first direction than a width dimension of a frame portion disposed at an end in the first direction.

6. The acoustic matching material supply device according to any one of claims 2 to 5,
wherein the plurality of frame portions are provided in an array form along a first direction and a second direction intersecting the first direction.

7. The acoustic matching material supply device according to claim 6,
wherein the plurality of frame portions include attaching/detaching mechanisms that allow the frame portion to be attached to or detached from the ultrasonic probe so that the inner peripheral surfaces avoid a plurality of ultrasonic measurement regions with respect to the ultrasonic sensor surface in which the ultrasonic measurement regions are disposed in an array form along the first direction and the second direction.

8. The acoustic matching material supply device according to claim 6,
wherein, among the plurality of frame portions, a frame portion located at the center has a smaller width dimension along at least one of the first direction and the second direction than a width dimension of a frame portion disposed at an outer periphery.

9. The acoustic matching material supply device according to any one of the preceding claims,
wherein the frame portion includes a protrusion that protrudes from the inner peripheral surface toward the center and has a branching passage communicating with the flow passage, and
wherein the protrusion is provided with a second ejection port that communicates with the branching passage and ejects the acoustic matching material to the outside on a side surface which intersects the ultrasonic sensor surface and faces the ultrasonic sensor surface.

10. The acoustic matching material supply device according to any one of the preceding claims, further comprising:
a deaeration passage that discharges a gas contained in an ejected acoustic matching material which is ejected from the frame portion, to the outside.

11. The acoustic matching material supply device according to claim 10,
wherein the frame portion is provided with a notch portion formed by notching a part of the frame portion, and
wherein the deaeration passage is provided at the notch portion.

12. The acoustic matching material supply device according to any one of the preceding claims, further comprising:
an opening/closing portion that opens and closes a flow passage between the ejection port and the introduction port.

13. The acoustic matching material supply device according to claim 12, further comprising:
a control unit that controls the opening/closing portion,
wherein the control unit includes
a region detection portion that detects an insufficiency region in which the acoustic matching material is insufficient, and a sufficiency region in which the acoustic matching material is sufficient, and
an opening/closing control portion that opens the opening/closing portion corresponding to an ejection port provided in the insufficiency region, and closes the opening/closing portion corresponding to an ejection port provided in the sufficiency region.

14. The acoustic matching material supply device according to claim 13,
wherein the region detection portion detects the insufficiency region and the sufficiency region on the basis of a received signal acquired by transmitting the ultrasonic waves to a target object from the ultrasonic probe and receiving the ultrasonic waves which are reflected from the target object.

15. An acoustic matching material supply device comprising:
a frame portion that is attached to an ultrasonic probe including an ultrasonic sensor surface for performing at least one of transmission and reception of ultrasonic waves, and ejects an acoustic matching material,
wherein the frame portion includes
a flow passage forming portion in which a flow passage of an acoustic matching material is formed, and
an introduction port that introduces the acoustic matching material into the flow passage of the flow passage forming portion, and
wherein the flow passage forming portion has a central extending portion which extends toward the center of the ultrasonic sensor surface, and the central extending portion is provided with a central ejection port which ejects the acoustic matching material toward the center of the ultrasonic sensor surface.

16. An ultrasonic probe unit comprising:
the acoustic matching material supply device according to any one of the preceding claims; and
the ultrasonic probe.

17. An ultrasonic measurement apparatus comprising:
the ultrasonic probe unit according to claim 16; and
a control device that controls the ultrasonic probe.

18. An ultrasonic image display comprising:
the ultrasonic measurement apparatus according to claim 17; and
a display device that displays an image,
wherein the control device generates an internal tomographic image of a target object on the basis of ultrasonic measurement using the ultrasonic probe, and displays the ultrasonic image on the display device.
